(19) **Europäisches Patentamt / European Patent Office / Office européen des brevets**

(11) **EP 4 560 441 A1**

(12) # EUROPEAN PATENT APPLICATION

(43) Date of publication:
28.05.2025 Bulletin 2025/22

(21) Application number: 24206457.4

(22) Date of filing: **14.10.2024**

(51) International Patent Classification (IPC):
**G06F 3/01** (2006.01)    **A63B 21/015** (2006.01)
**A63B 71/00** (2006.01)    **A63B 22/02** (2006.01)
**A63B 24/00** (2006.01)    **A63B 71/06** (2006.01)
**A63B 69/16** (2006.01)

(52) Cooperative Patent Classification (CPC):
**A63B 22/0292; A63B 21/015; A63B 24/0087;
A63B 71/0009; A63B 71/0619; G06F 3/0362;
G06F 3/038;** A63B 2024/009; A63B 2024/0093;
A63B 2024/0096; A63B 2069/167; A63B 2071/0018;
A63B 2209/08; A63B 2220/24; A63B 2220/30;
(Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **21.11.2023   KR 20230162389
14.06.2024   KR 20240077789**

(71) Applicant: **Kangsters Corp.
Seongnam-si Gyeonggi-do 13449 (KR)**

(72) Inventors:
• **KIM, Kang**
**15337 Ansan-si (KR)**
• **LEE, Yoonsang**
**15580 Ansan-si (KR)**
• **CHOI, Chiheon**
**15340 Ansan-si (KR)**
• **BAEK, Seungsoo**
**18273 Hwaseong-si (KR)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(54) ## ELECTRONIC DEVICE COMMUNICATING WITH TREADMILL FOR WHEELCHAIR

(57)    The present invention relates to an electronic device including: a communication module communicating with a treadmill for a wheelchair; a display for outputting a screen of an application; and a processor, wherein in an input mode, the processor may acquire first data representing the rotation of a first roller structure of the treadmill for the wheelchair and second data representing the rotation of a second roller structure of the treadmill for the wheelchair through the communication module, analyze the first data and the second data to convert the analyzed data into data related to the rotation of a first main wheel and data related to the rotation of a second main wheel of the wheelchair placed on top of the treadmill for the wheelchair, match the data related to the rotation of the first main wheel and the data related to the rotation of the second main wheel to an input command, and control the application according to the input command.

FIG. 9A

Start
↓
Conversion to input mode — 910
↓
Acquisition of first data and second data from a treadmill through a communication module — 920
↓
Analysis of first data and second data and conversion of the analyzed data into data related to the rotations of main wheels — 930
↓
Matching the data related to the rotation of the first main wheel and the data related to the rotation of the second main wheel to an input command — 940
↓
Control of application according to the input command — 950
↓
End

**(Cont. next page)**

(52) Cooperative Patent Classification (CPC): (Cont.)
A63B 2220/51; A63B 2220/62; A63B 2225/20;
A63B 2225/50

**Description**

## BACKGROUND OF THE DISCLOSURE

### Cross Reference to Related Application of the Disclosure

The present application claims the benefit of Korean Patent Application No.10-2023-0162389 filed in the Korean Intellectual Property Office on November 21, 2023, and Korean Patent Application No.10-2024-0077789 filed in the Korean Intellectual Property Office on June 14, 2024, the entire contents of which are incorporated herein by reference.

### Field of the Disclosure

The present invention relates to an electronic device communicating with a treadmill for a wheelchair, more specifically to an electronic device communicating with a treadmill for a wheelchair that is capable of providing a user's workout information.

### Background of the Related Art

A wheelchair is one of the most commonly used assistive devices to promote mobility and enhance quality of life for people who have difficulties in walking. If a wheelchair user uses his or her wheelchair for a long period of time, he or she may feel his or her physical strength decline because of the lack of exercise. To solve such a problem, there has been a lot of interest in a treadmill for a wheelchair on which a wheelchair user works out using the wheelchair placed thereon.
However, in the case of the conventional treadmill for a wheelchair, unlike existing treadmills, a wheelchair user works out in a state where he or she sits in the wheelchair, so that it is impossible to measure the information of an amount of his or her workout. If the information of the amount of the user's workout is not obtained, the content capable of giving motivation for the user's consistent workout is not enough, thereby failing to provide good workout effectiveness for the user who uses the conventional treadmill for a wheelchair.

## SUMMARY OF THE DISCLOSURE

Accordingly, the present disclosure has been made in view of the above-mentioned problems occurring in the related art, and it is an object of the present disclosure to provide an electronic device communicating with a treadmill for a wheelchair that is capable of providing a user's workout information.
To accomplish the above-mentioned objects, according to an aspect of the present disclosure, there is provided an electronic device including: a communication module communicating with a treadmill for a wheelchair; a display for outputting a screen of an application; and a processor, wherein in an input mode, the processor may acquire first data representing the rotation of a first roller structure of the treadmill for the wheelchair and second data representing the rotation of a second roller structure of the treadmill for the wheelchair from the treadmill for the wheelchair through the communication module, analyze the first data and the second data to convert the analyzed data into data related to the rotation of a first main wheel and data related to the rotation of a second main wheel of the wheelchair placed on top of the treadmill for the wheelchair, match the data related to the rotation of the first main wheel and the data related to the rotation of the second main wheel to an input command, and control the application according to the input command.
According to the embodiment of the present disclosure, the data related to the rotation of the first main wheel may include a rotational direction of the first main wheel, the data related to the rotation of the second main wheel may include a rotational direction of the second main wheel, and the processor may match a forward direction as the rotational direction of the first main wheel and a forward direction as the rotational direction of the second main wheel to a first input command, match the forward direction as the rotational direction of the first main wheel and a backward direction as the rotational direction of the second main wheel to a second input command, match a backward direction as the rotational direction of the first main wheel and the forward direction as the rotational direction of the second main wheel to a third input command, match the backward direction as the rotational direction of the first main wheel and the backward direction as the rotational direction of the second main wheel to a fourth input command, match the forward direction as the rotational direction of the first main wheel and no rotation of the second main wheel to a fifth input command, match the backward direction as the rotational direction of the first main wheel and no rotation of the second main wheel to a sixth input command, match no rotation of the first main wheel and the forward direction as the rotational direction of the second main wheel to a seventh input command, and match no rotation of the first main wheel and the backward direction as the rotational direction of the second main wheel to an eighth input command.
According to the embodiment of the present disclosure, the data related to the rotation of the first main wheel may include a rotational angle of the first main wheel, the data related to the rotation of the second main wheel may include a rotational angle of the second main wheel, the fifth input command may be a command that moves a cursor in a first direction, based on the rotational angle of the first main wheel, the sixth input command may be a command that moves the cursor in a second direction, based on the rotational angle of the first main wheel, the seventh input command may be a command that moves the cursor in a third direction, based on the rotational angle of the second main wheel, and the eighth input command may be a command that moves the cursor in a fourth direction, based on the rotational angle of the second main wheel.

According to the embodiment of the present disclosure, the processor may execute a training mode according to the input command, acquire the first data and the second data from the treadmill for the wheelchair through the communication module, analyze the first data and the second data to convert the analyzed data into the data related to the rotation of the first main wheel and the data related to the rotation of the second main wheel, and analyze a user's workout, based on the data related to the rotation of the first main wheel and the data related to the rotation of the second main wheel.

According to the embodiment of the present disclosure, the processor may collect the user's workout analysis data in the training mode, evaluate the user's workout ability, based on the user's workout analysis data, and produce a customized training mode using an artificial intelligence model, based on the user's workout ability data evaluated.

According to the embodiment of the present disclosure, if the value of the user's workout ability data for a given workout is less than an average data value, the processor may produce the customized training mode for training the user in strengthening his or her workout ability with the data value less than the average data value.

According to the embodiment of the present disclosure, if the value of the user's workout ability data is greater than or equal to the average data value, the processor may produce the customized training mode for training the user in strengthening the user's overall workout ability, based on the history of the user's workout ability data.

According to the embodiment of the present disclosure, the processor may execute the produced customized training mode, collect the user's workout analysis data in the customized training mode to evaluate the user's workout ability, and update the artificial intelligence model, based on the user's workout analysis data evaluated in the customized training mode.

According to the embodiment of the present disclosure, the processor may execute a game mode according to the input command, acquire the first data and the second data from the treadmill for the wheelchair through the communication module, analyze the first data and the second data to convert the analyzed data into the data related to the rotation of the first main wheel and the data related to the rotation of the second main wheel, match the data related to the rotation of the first main wheel and the data related to the rotation of the second main wheel to the input command, and control a game character in the game mode according to the input command.

According to the embodiment of the present disclosure, if the game mode is a running game, the processor may match a forward direction as the rotational direction of the first main wheel and a forward direction as the rotational direction of the second main wheel to a forward movement command of the game character, match the forward direction as the rotational direction of the first main wheel and a backward direction as the rotational direction of the second main wheel to a movement command to a +z-axis of the game character, match a backward direction as the rotational direction of the first main wheel and the forward direction as the rotational direction of the second main wheel to a movement command to a -z-axis of the game character, match the backward direction as the rotational direction of the first main wheel and the backward direction as the rotational direction of the second main wheel to a backward movement command of the game character, match the forward direction as the rotational direction of the first main wheel and no rotation of the second main wheel to a forward right turn command of the game character, match the backward direction as the rotational direction of the first main wheel and no rotation of the second main wheel to a backward right turn command of the game character, match no rotation of the first main wheel and the forward rotation as the rotational direction of the second main wheel to a forward left turn command of the game character, and match no rotation of the first main wheel and the backward rotation as the rotational direction of the second main wheel to a backward left turn command of the game character, the processor determining a moving speed of the game character, based on a rotational speed of the first main wheel and/or a rotational speed of the second main wheel.

## BRIEF DESCRIPTION OF THE DRAWINGS

The present disclosure is capable of undergoing various modifications and may have numerous embodiments. Specific embodiments are illustrated in the drawings and will be described in detail in the following description. The effects and features of the present disclosure, and the methods of achieving them, will become apparent with reference to the embodiments described in detail below along with the accompanying drawings. However, the present disclosure is not limited to the embodiments described below but may be embodied in various forms.

In the following embodiments, terms such as 'first,' 'second,' etc., are used to distinguish one component from another and are not intended to be limiting. In the following embodiments, singular terms include plural forms unless explicitly stated otherwise in the context. In the following embodiments, terms such as 'include' or 'have' indicate the presence of the stated features or components and do not preclude the possibility of additional features or components.

In the following embodiments, when a part such as a layer, region, or component is described as being 'on' or 'above' another part, this includes both cases where the part is directly on top of the other part, and cases where there is an intervening region or component in between. In the drawings, for convenience of explanation, the sizes of components may be exaggerated or reduced. For instance, the sizes and thicknesses of the components shown in the drawings are illustrated arbitrarily for the sake of explanation, and the present disclosure is not limited to the illustrated aspects.

In some embodiments, when a specific sequence of operations is described, the sequence may be performed in a different order than described. For example, two sequential steps may be performed substantially simultaneously or in the reverse order of the described sequence.

In this specification, "A and/or B" refers to cases where A, B, or both A and B are present. Also, "at least one of A and B" refers to cases where A, B, or both A and B are present.

In the following embodiments, when a layer, region, or component is described as being 'connected' to another, this includes both direct connections and indirect connections through other layers, regions, or components. For example, when it is stated in this specification that a layer, region, or component is electrically connected, it means that the layer, region, or component is either directly electrically connected or indirectly electrically connected through other intervening layers, regions, or components.

The x-axis, y-axis, and z-axis are not limited to the three axes in a Cartesian coordinate system but may be interpreted broadly to include them. For example, the x-axis, y-axis, and z-axis may be orthogonal to each other, but they may also refer to different directions that are not orthogonal.

The advantages and features of the present disclosure, and the methods of achieving them, will become apparent with reference to the embodiments described in detail below along with the accompanying drawings. However, the present disclosure is not limited to the embodiments described below and may be implemented in various other forms. These embodiments are provided to make the disclosure complete and to fully convey the scope of the disclosure to those skilled in the art, and the present disclosure is defined only by the claims.

The terms used in the present disclosure are for the purpose of describing the embodiments and are not intended to limit the disclosure. In the present disclosure, the singular form may include the plural form unless otherwise specified. The terms "comprises" and/or "comprising," as used throughout the disclosure, do not exclude the presence or addition of one or more other features or components in addition to the mentioned features or components. Throughout the disclosure, the same reference numerals refer to the same components, and "and/or" may include each and any combination of the mentioned components. Although terms like 'first,' 'second,' etc., are used to describe various components, these components are not limited by these terms. These terms are used only to distinguish one component from another. Thus, a first component mentioned below could also be a second component within the technical spirit of the present disclosure.

The term "exemplary" as used in the present disclosure means "used as an example or illustration." Any embodiment described as "exemplary" in the present disclosure should not be construed as being preferred or having advantages over other embodiments.

The embodiments of the present disclosure may be described in terms of functions or functional blocks that perform certain actions. The blocks, which may be referred to as "units" or "modules" in the present disclosure, can be physically implemented using analog or digital circuits such as logic gates, integrated circuits, microprocessors, microcontrollers, memory, passive electronic components, active electronic components, optical components, hardwired circuits, and the like, and may optionally be operated by firmware and software. Furthermore, the term "unit" as used in the present disclosure may refer to hardware elements such as software, FPGA, or ASIC, and the "unit" can perform various roles. However, the "unit" is not limited to software or hardware. The "unit" may be configured to exist on an addressable storage medium and to reproduce one or more processors. Therefore, by way of example, the "unit" may include elements such as software elements, object-oriented software elements, class elements, and task elements, as well as processes, functions, attributes, procedures, subroutines, segments of program code, drivers, firmware, microcode, circuits, data, databases, data structures, tables, arrays, and variables. The functionality provided within the elements and "units" can be combined into fewer elements and "units" or further separated into additional elements and "units".

The embodiments of the present disclosure may be implemented using at least one software program executed on at least one hardware device and may perform network management functions to control the elements.

Spatially relative terms such as "below," "beneath," "lower," "above," "upper," and the like can be used to describe the relationship between one component and other components as illustrated in the drawings, to facilitate description. Spatially relative terms are to be understood as including different orientations in use or operation in addition to the direction shown in the drawings. For example, if a component depicted in the drawings is turned over, components described as "below" or "beneath" other components would then be oriented "above" the other components. Thus, the exemplary term "below" can encompass both upward and downward directions. Components may be oriented in different ways, and accordingly, spatially relative terms may be interpreted according to orientation.

Unless otherwise defined, all terms (including technical and scientific terms) used herein have the same meaning as commonly understood by one of ordinary skill in the art to which this disclosure belongs. Moreover, the terms defined in commonly used dictionaries should be interpreted as having a meaning that is consistent with their meaning in the context of the relevant art, and should not be interpreted in an idealized or overly formal sense unless expressly defined herein.

Hereinafter, embodiments of the present disclosure will be described in detail with reference to the accompanying drawings, and when describing the drawings, the same or corresponding components will be denoted by the same reference numerals, and redundant descriptions thereof will be omitted.

The above and other objects, features and advantages of the present disclosure will be apparent from the following detailed description of the embodiments of the disclosure in conjunction with the accompanying drawings, in which:

FIG. 1 is a schematic perspective view showing a treadmill for a wheelchair according to an exemplary embodiment of the present disclosure;

FIG. 2 is a schematic perspective view showing a state where the wheelchair is located on the treadmill for the wheelchair according to the exemplary embodiment of the present disclosure;

FIG. 3 is a schematic sectional view showing a first roller structure of the treadmill for the wheelchair according to the exemplary embodiment of the present disclosure;

FIG. 4 is a schematic top view showing a portion of the treadmill for the wheelchair according to the exemplary embodiment of the present disclosure;

FIG. 5 is a schematic perspective view showing an example of a relation between a first operating part and a first sensing part of the treadmill for the wheelchair according to the exemplary embodiment of the present disclosure;

FIG. 6 is a schematic perspective view showing another example of the relation between the first operating part and the first sensing part of the treadmill for the wheelchair according to the exemplary embodiment of the present disclosure;

FIG. 7 is a schematic perspective view showing yet another example of the relation between the first operating part and the first sensing part of the treadmill for the wheelchair according to the exemplary embodiment of the present disclosure;

FIG. 8 is a schematic block diagram showing a system having the treadmill for the wheelchair according to the exemplary embodiment of the present disclosure;

FIG. 9A is a schematic flowchart showing an example of operations of an electronic device according to the exemplary embodiment of the present disclosure;

FIG. 9B is a schematic view showing examples of operations related to rotations of main wheels according to the exemplary embodiment of the present disclosure;

FIG. 9C is a schematic view showing an example related to rotational angles of the main wheels according to the exemplary embodiment of the present disclosure;

FIGs. 9D to 9F are schematic views showing examples of operations of the electronic device for controlling an application according to matched input commands according to the exemplary embodiment of the present disclosure;

FIG. 10A is a schematic flowchart showing an example of operations of the electronic device according to the exemplary embodiment of the present disclosure;

FIG. 10B is a schematic view showing an example of a training mode of the electronic device according to the exemplary embodiment of the present disclosure;

FIG. 11A is a schematic flowchart showing an example of operations of the electronic device according to the exemplary embodiment of the present disclosure;

FIG. 11B is a schematic view showing an example of a training mode result according to the exemplary embodiment of the present disclosure;

FIG. 11C is a schematic view showing an example of a customized training mode according to the exemplary embodiment of the present disclosure;

FIG. 12A is a schematic flowchart showing an example of operations of the electronic device according to the exemplary embodiment of the present disclosure;

FIG. 12B is a schematic view showing an example of a game application screen provided by the electronic device according to the exemplary embodiment of the present disclosure; and

FIG. 12C is a schematic view showing another example of the game application screen provided by the electronic device according to the exemplary embodiment of the present disclosure.

## DETAILED DESCRIPTION OF THE PREFERRED EMBODIMENT

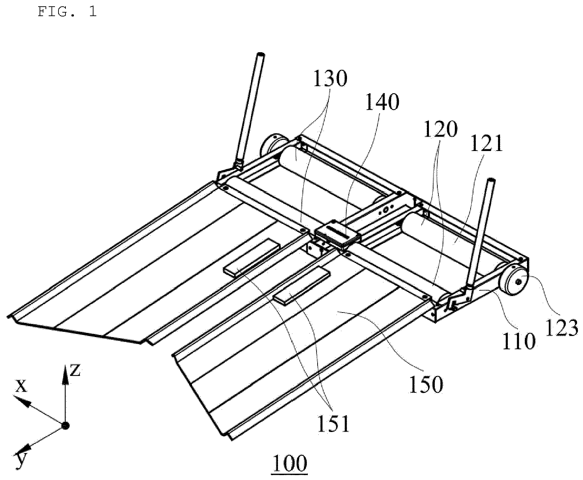

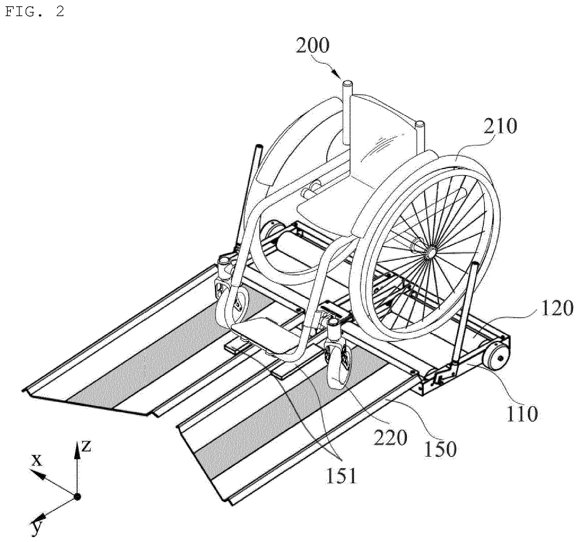

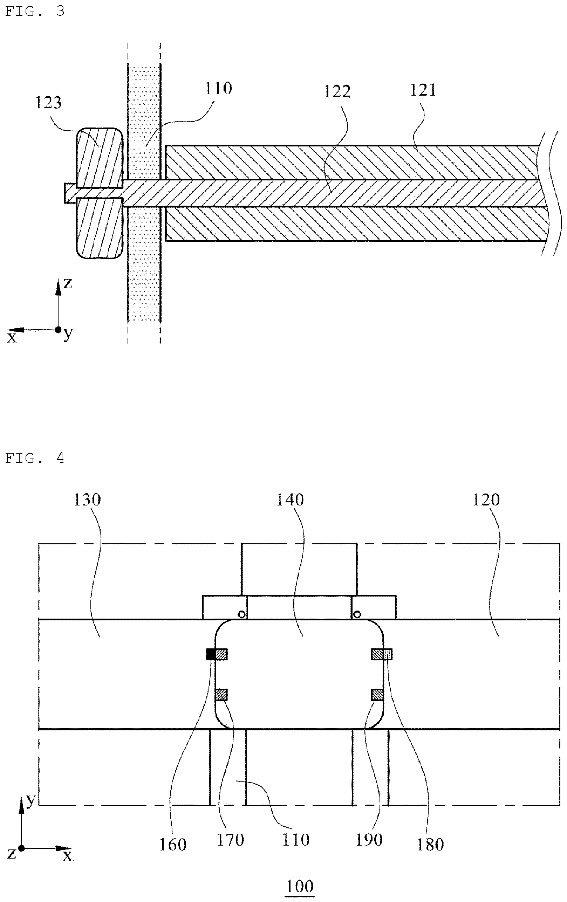

FIG. 1 is a schematic perspective view showing a treadmill for a wheelchair according to an exemplary embodiment of the present disclosure, FIG. 2 is a schematic perspective view showing a state where the wheelchair is located on the treadmill for the wheelchair according to the exemplary embodiment of the present disclosure, and FIG. 3 is a schematic sectional view showing a first roller structure of the treadmill for the wheelchair according to the exemplary embodiment of the present disclosure.

Referring to FIGs. 1 to 3, a treadmill 100 for a wheelchair 200 according to an exemplary embodiment of the present disclosure includes a first frame 110, a first roller structure 120, a second roller structure 130, a communication module 140, and a second frame 150.

The first frame 110 supports at least a portion of the load applied to the treadmill 100 for the wheelchair 200. The first frame 110 serves to support main wheels 210 of the wheelchair 200 located on the treadmill 100. The first frame 110 is located on the floor. For example, the first frame 110 is made of a relatively heavy material (e.g., metal) so that while a user is working out, the treadmill 100 for the wheelchair 200 does not move in position.

The first roller structure 120 is rotatably coupled to the first frame 110. For example, the first roller structure 120 is located inside the first frame 110 in such a way as to be surrounded by the first frame 110. The first roller structure 120 is rotatable by means of the rotation of one of the main wheels 210 of the wheelchair 200. For example, the first roller structure 120 has a circular cross-section so that it can rotate smoothly with respect to the first frame 110. The first roller structure 120 extends in a first direction (e.g., in a direction of +x-axis) and thus has the shape of a cylinder with a given length in the first direction. According to an embodiment of the present disclosure, the first roller structure 120 has a plurality of rollers. The plurality of rollers are spaced apart from each other in a second direction (e.g., in a direction of +y- axis) transversing the first direction. While the wheelchair 200 is being located on the treadmill 100, one of the main wheels 210 of the wheelchair 200 is located(or received) in a space between the plurality of rollers.

The second roller structure 130 is rotatably coupled to the first frame 110. For example, the second roller structure 130 is located inside the first frame 110 in such a way as to be surrounded by the first frame 110. The second roller structure 130 is rotatable by means of the rotation of the other of the main wheels 210 of the wheelchair 200. For example, the second roller structure 130 has a circular cross-section so that it can rotate smoothly with respect to the first frame 110. The second roller structure 130 extends in the first direction (e.g., in the direction of +x-axis) and thus has the shape of a cylinder with a given length in the first direction. According to an embodiment of the present disclosure, the second roller structure 130 has a plurality of rollers. The plurality of rollers are spaced apart from each other in the second direction (e.g., in the direction of +y- axis) transversing the first direction. While the wheelchair 200 is being located on the treadmill 100, the other of the main wheels 210 of the wheelchair 200 is located(or received) in the space between the plurality of rollers. The second roller structure 130 is spaced apart from the first roller structure 120 in the first direction (e.g., in the direction of +x-axis).

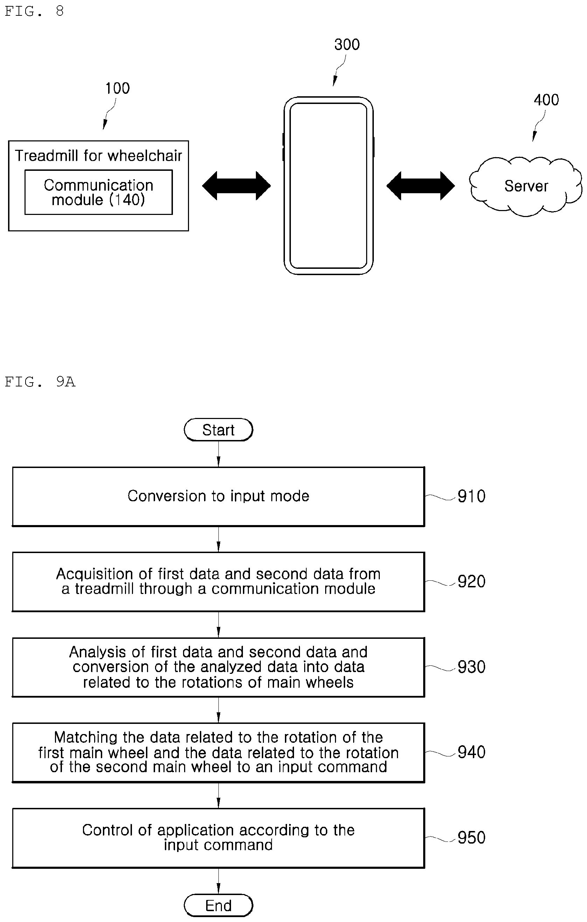

The communication module 140 serves to perform the communication between the treadmill 100 for the wheelchair 200 and an external electronic device 300 (See FIG. 8). For example, the communication module 140 transmits the data acquired by the treadmill 100 for the wheelchair 200 to the electronic device 300 or receives data from the electronic device 300. According to an embodiment of the present disclosure, the communication module 140 performs wireless communication with the electronic device 300. For example, the communication module 140 performs wireless communication with the electronic device 300 through various communication technologies, such as a cellular communication technology, a Wi-Fi communication technology, an NFC technology, and a Bluetooth communication technology. The communication module 140 is located between the first roller structure 120 and the second roller structure 130.

The second frame 150 serves to support auxiliary wheels 220 of the wheelchair 200. The second frame 150 is brought into close contact with the auxiliary wheels 220 of the wheelchair 200. The second frame 150 is coupled to the first frame 110. For example, the second frame 150 extends in the second direction (e.g., in the direction of +y-axis). For example, the second frame 150 has a plurality of plates spaced apart from each other by a given distance, but it may not be limited thereto. According to an embodiment of the present disclosure, the second frame 150 has a plurality of guides 151. The second frame 150 is referred to as (or is called) a footboard.

The plurality of guides 151 serve to prevent (or suppress) the wheelchair 200 from rotating while the user is working out using the treadmill 100 for the wheelchair 200. The plurality of guides 151 are located between the auxiliary wheels 220 of the wheelchair 200 while the wheelchair 200 is being located on the treadmill 100 for the wheelchair 200. As the rotation of the wheelchair 200 is limited by means of the plurality of guides 151, the user of the treadmill 100 for the wheelchair 200 works out in a safe manner. The plurality of guides 151 are spaced apart from each other. For example, the plurality of guides 151 are spaced apart from each other in the first direction (e.g., in the direction of +x-axis). For example, a distance between the plurality of guides 151 is shorter than a distance between the auxiliary wheels 220. The plurality of guides 151 are located (formed) on the second frame 150. For example, the plurality of guides 151 protrude from one surface of the second frame 150 coming into contact with the auxiliary wheels 220. For example, the plurality of guides 151 protrude from one surface of the second frame 150 in a third direction (e.g., in a direction of +z-axis) perpendicular to both of the first direction (e.g., in the direction of +x-axis) and the second direction (e.g., in the direction of +y-axis).

According to an embodiment of the present disclosure, the first roller structure 120 includes a roller 121, a shaft 122, and a holder 123. The first roller structure 120 will be described below, but the embodiment of the present disclosure may not be limited thereto. For example, an explanation of the first roller structure 120 may be given in the same manner as the second roller structure 130. The roller 121 is rotatable with respect to the first frame 110. The roller 121 rotates by means of the rotations of the main wheels 210. The shaft 122 passes through the roller 121 and the first frame 110. The shaft 122 is rotatable together with the roller 121 so that it is rotatable relative to the first frame 110. The holder 123 is coupled to the shaft 122 to apply the moment of rotational inertia to the roller 121. As the shaft 122 passes through the holder 123, the holder 123 is rotatable together with the shaft 122 and the roller 121. The moment of inertia of the first roller structure 120 becomes relatively higher through the holder 123 when compared to the case where no holder 123 exists. As the moment of inertia of the first roller structure 120 becomes relatively high, the rotation of the first roller structure 120 is kept relatively longer. As the rotation of the first roller structure 120 becomes relatively long, the user who is physically disabled can work out easily. When the first roller structure 120 is seen in an extension direction of the shaft 122, the cross-sectional area of the holder 123 is larger than the cross-sectional areas of the shaft 122 and the roller 121. For example, the cross-sectional area of the holder 123 has the shape of a circle, but the present disclosure may not be limited thereto. That is, the cross-sectional area of the holder 123 has the shape of an oval and/or polygon.

FIG. 4 is a schematic top view showing a portion of the treadmill for the wheelchair according to the exemplary embodiment of the present disclosure.

Referring to FIG. 4, the treadmill 100 for the wheelchair 200 includes a first operating part 160, a first sensor part 170, a second operating part 180, and a second sensor part 190.

The first operating part 160 is coupled to the first roller structure 120 in such a way as to be rotatable together with the first roller structure 120. For example, the first operating part 160 is located (or inserted) inside the first roller structure 120, but the present disclosure may not be limited thereto. For example, the first operating part 160 is exposed to the outside of the first roller structure 120. For example, the first operating part 160 is coupled to one end of both ends of the first roller structure 120 that is close to the second roller structure 130 (or the communication module 140).

The first sensor part 170 is located on the first frame 110. The first sensor part 170 interacts with the first operating part 160 to acquire first data representing the rotation of the first roller structure 120. The first sensor part 170 is oriented toward the first operating part 160. For example, if the first operating part 160 is a magnetic material (or magnet), the first sensor part 170 interacts with the first operating part 160, based on a magnetic field, and thus acquires the first data. However, the present disclosure may not be limited thereto.

The second operating part 180 is coupled to the second roller structure 130 in such a way as to be rotatable together with the second roller structure 130. For example, the second operating part 180 is located (or inserted) inside the second roller structure 130, but the present disclosure may not be limited thereto. For example, the second operating part 180 is exposed to the outside of the second roller structure 130. For example, the second operating part 180 is coupled to one end of both ends of the second roller structure 130 that is close to the first roller structure 120 (or the communication module 140).

The second sensor part 190 is located on the first frame 110. The second sensor part 190 interacts with the second operating part 180 to acquire second data representing the rotation of the second roller structure 130. The second sensor part 190 is oriented toward the second operating part 180. For example, if the second operating part 180 is a magnetic material (or magnet), the second sensor part 190 interacts with the second operating part 180, based on a magnetic field, and thus acquires the second data. However, the present disclosure may not be limited thereto.

According to an embodiment of the present disclosure, the communication module 140 transmits at least one of the first data acquired from the first sensor part 170 and the second data acquired from the second sensor part 190 to the external electronic device 300 (See FIG. 8) that displays the workout of the user using the treadmill 100 for the wheelchair 200. The first data include the rotational speed and direction of the first roller structure 120, and the second data include the rotational speed and direction of the second roller structure 130. A method for acquiring the first data through the first operating part 160 and the first sensor part 170 will be explained below for the convenience of the description. The method for acquiring the first data through the first operating part 160 and the first sensor part 170 may be applied in the same manner as a method for acquiring the second data through the second operating part 180 and the second sensor part 190.

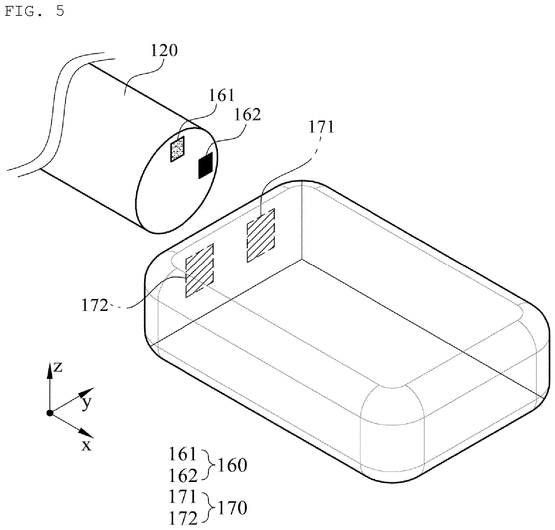

FIG. 5 is a schematic perspective view showing an example of a relation between the first operating part and the first sensing part of the treadmill for the wheelchair according to the exemplary embodiment of the present disclosure.

Referring to FIG. 5, the first operating part 160 includes a plurality of magnets 161 and 162. The plurality of magnets 161 and 162 have different magnetism from each other. For example, the magnetism of the first magnet 161 is stronger than that of the second magnet 162. The plurality of magnets 161 and 162 are asymmetrically arranged around the center of the first roller structure 120. For example, the plurality of magnets 161 and 162 are located on the first quadrant of the first roller structure 120 around the center of the first roller structure 120, but they may not be limited thereto.

The first sensor part 170 includes at least one or more magnetic sensors 171 and 172. The at least one or more magnetic sensors 171 and 172 are referred to as hall sensors. For example, the first magnetic sensor 171 and the second magnetic sensor 172 are spaced apart from each other in the second direction (e.g., in the direction of +y).

The first and second magnetic sensors 171 and 172 acquire the first data for identifying the rotational direction of the first roller structure 120, based on a time interval between sections where a magnetic field is sensed through the plurality of magnets 161 and 162.

At least one of the first and second magnetic sensors 171 and 172 acquire the first data for identifying the rotational direction of the first roller structure 120, based on the order where the plurality of magnets 161 and 162 are sensed. For example, the first magnet 161 and the second magnet 162 are arranged in a clockwise direction, and if the first roller structure 120 rotates in the clockwise direction, the first magnetic sensor 171 acquires the first data having a section where the size of the magnetic field increases. For example, the first magnet 161 and the second magnet 162 are arranged in a clockwise direction, and if the first roller structure 120 rotates in a counterclockwise direction, the first magnetic sensor 171 acquires the first data having a section where the size of the magnetic field decreases.

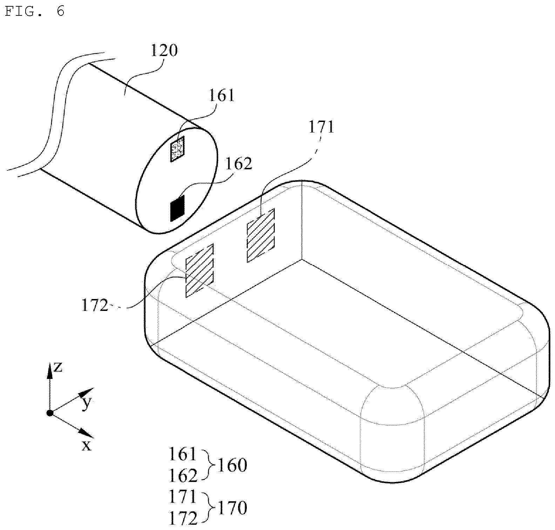

FIG. 6 is a schematic perspective view showing another example of the relation between the first operating part and the first sensing part of the treadmill for the wheelchair according to the exemplary embodiment of the present disclosure.

Referring to FIG. 6, the first operating part 160 includes a plurality of magnets 161 and 162. The plurality of magnets 161 and 162 have different magnetism from each other. For example, the magnetism of the first magnet 161 is stronger than that of the second magnet 162. The plurality of magnets 161 and 162 are symmetrically arranged around the center of the first roller structure 120. For example, the plurality of magnets 161 and 162 are located to face each other around the center of the first roller structure 120.

The first sensor part 170 includes at least one or more magnetic sensors 171 and 172. The at least one or more magnetic sensors 171 and 172 are referred to as hall sensors. For example, the first magnetic sensor 171 and the second magnetic sensor 172 are spaced apart from each other in the second direction (e.g., in the direction of +y).

The first and second magnetic sensors 171 and 172 acquire the first data for identifying the rotational direction of the first roller structure 120, based on a time interval between sections where a magnetic field is sensed through the plurality of magnets 161 and 162.

The first and second magnetic sensors 171 and 172 acquire the first data for identifying the rotational direction of the first roller structure 120, based on the order where the plurality of magnets 161 and 162 are sensed. For example, if the first roller structure 120 rotates in a clockwise direction, the first magnet 161 is first sensed through the first magnet sensor 171 before the second magnet 162 is sensed. The rotational direction of the first roller structure 120 is identified according to one of the plurality of magnets 161 and 162 that is first sensed through the plurality of first and second magnetic sensors 171 and 171.

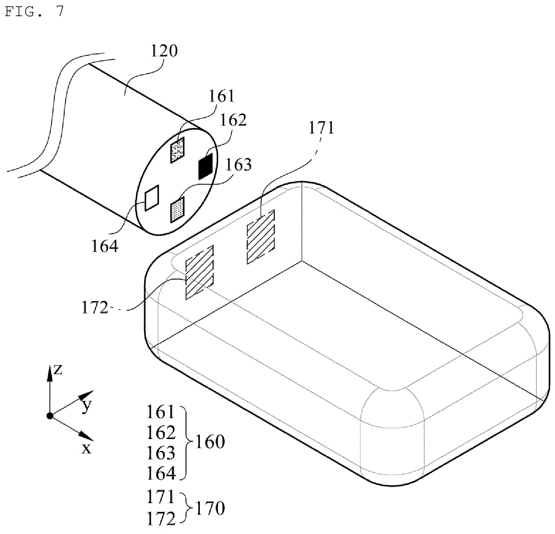

FIG. 7 is a schematic perspective view showing yet another example of the relation between the first operating part and the first sensing part of the treadmill for the wheelchair according to the exemplary embodiment of the present disclosure.

Referring to FIG. 7, the first operating part 160 includes a plurality of magnets 161, 162, 163, and 164. The plurality of magnets 161, 162, 163, and 164 have different magnetism from one another. For example, the magnetism of the first magnet 161 is stronger than that of the second magnet 162, the magnetism of the second magnet 162 is stronger than that of the third magnet 163, and the magnetism of the third magnet 163 is stronger than that of the fourth magnet 164. The plurality of magnets 161, 162, 163, and 164 are symmetrically arranged around the center of the first roller structure 120. For example, the first magnet 161 and the third magnet 163 are located to face each other around the center of the first roller structure 120, and the second magnet 162 and the fourth magnet 164 are located to face each other around the center of the first roller structure 120.

The first sensor part 170 includes at least one or more magnetic sensors 171 and 172. The at least one or more magnetic sensors 171 and 172 are referred to as hall sensors. For example, the first magnetic sensor 171 and the second magnetic sensor 172 are spaced apart from each other in the second direction (e.g., in the direction of +y).

The first and second magnetic sensors 171 and 172 acquire the first data for identifying the rotational direction of the first roller structure 120, based on a time interval between sections where a magnetic field is sensed through the plurality of magnets 161 and 162.

The first and second magnetic sensors 171 and 172 acquire the first data for identifying the rotational direction of the first roller structure 120, based on the order where the plurality of magnets 161, 162, 163, and 164 are sensed. For example, if the first roller structure 120 rotates in a clockwise direction, the first magnetic sensor 171 acquires the first data having a section where the size of the magnetic field increases. For example, if the first roller structure 120 rotates in a counterclockwise direction, the first magnetic sensor 171 acquires the first data having a section where the size of the magnetic field decreases.

As described above, the treadmill for the wheelchair according to the embodiment of the present disclosure is configured to easily acquire the data for identifying the rotational speed and direction of the first roller structure 120 through the arrangement of the first operating part 160 and the first sensor part 170. Now, a method for providing content for the user through the first data representing the rotation of the first roller structure 120 and the second data representing the rotation of the second roller structure 130 will be explained.

FIG. 8 is a schematic block diagram showing a system having the treadmill for the wheelchair according to the exemplary embodiment of the present disclosure.

Referring to FIG. 8, a system having the treadmill 100 for the wheelchair according to an embodiment of the present disclosure includes the treadmill 100 for the wheelchair 200, the electronic device 300, and a server 400. However, the present disclosure may not be limited thereto. For example, either the electronic device 300 or the server 400 may not exist in the system having the treadmill 100 for the wheelchair.

According to an embodiment of the present disclosure, the electronic device 300 includes a processor, a display, and/or a communication module.

The operations of the electronic device 300 as mentioned above are performed through the processor of the electronic device 300.

The communication module of the electronic device 300 communicates with the treadmill 100 for the wheelchair 200 through Bluetooth and transmits and receives information to and from the treadmill 100.

According to an embodiment of the present disclosure, the treadmill 100 transmits at least one of the first data and the second data to the electronic device 300. The first data and the second data, which are based on a binary code, are represented. For example, at least one of the first data and the second data is represented as the following Table 1.

[Table 1]

| | Header | | Battery volt | | Measure Time | | | | L-Wheel Pos (pulses) | | | | R-Wheel Pos (pulses) | | | |
|---|---|---|---|---|---|---|---|---|---|---|---|---|---|---|---|---|
| Byte Index | 0 | 1 | 2 | 3 | 4 | 5 | 6 | 7 | 8 | 9 | 10 | 11 | 12 | 13 | 14 | 15 |
| Value | 0x5A5A | | 3000 0x0BB8 | | 1234 0x04D2 | | | | 10000 0xoooo2710 | | | | -10000 0xFFFFD8F0 | | | |
| Example | 0x5A | 0x5A | Ox B8 | Ox B8 | 0x D2 | Ox 04 | 0x 00 | Ox 00 | 0x 10 | 0x 27 | 0x 00 | 0x 00 | 0x F0 | 0x D8 | 0x FF | 0x FF |

In the above Table 1, the header is utilized as the reference for determining start of data when the binary code is transmitted. Further, the Battery Volt represents a battery voltage mV, and the Measure Time represents a measure time of 250 ms. The Wheel Pos represents the position of a wheel. That is, the Wheel Pos represents the positions of wheels through the number of times the operating parts 160 and 180 are sensed by the sensing parts 170 and 180. The first data or the second data exemplarily represented in Table 1 is transmitted to the electronic device 300 every 250 ms, that is, four times in one second. The electronic device 300 acquires the workout information of the user of the treadmill 100, based on the first data and the second data received. The electronic device 300 performs the operation for the first data and the second data through the application processor built therein. For example, the electronic device 300 acquires the information of the user's workout through the following mathematical expressions.

Workout distance = 2 nr (Current Wheel pos - Previous Wheel pos)/(the number of magnets of the roller structure)

In this case, a reference symbol r in Mathematical expression 1 represents the radius of one of the first roller structure 120 and the second roller structure 130.

Workout time = (Current Measure time - Previous Measure time) * Data transmission period

For example, if the data transmission period is 250 ms in Mathematical expression 2, 0.25 is inputted to the data transmission period in Mathematical expression 2.

$$[\text{Mathematical expression 3}]$$

$$\text{Speed (km/h)} = (\text{Distance/Time}) * 3.6$$

Further, the operation for the first data and the second data may be performed through the server 400 that receives the first data and the second data from the electronic device 300.

The operations of the electronic device 300 as will be described below with reference to FIGs. 9A to 12B may be performed through the processor of the electronic device 300.

FIG. 9A is a schematic flowchart showing an example of the operations of the electronic device 300 according to the exemplary embodiment of the present disclosure.

According to various embodiments of the present disclosure, in step 910, the electronic device 300 allows the application to be converted to be in an input mode.

For example, the input mode is a mode that selects at least one of programs of the application. For example, the input mode is a mode that inputs characters in the application. For example, the input mode is a mode that selects a menu in the application.

According to various embodiments of the present disclosure, in step 920, the electronic device 300 acquires the first data and the second data from the treadmill 100 for the wheelchair 200 through the communication module.

According to an embodiment of the present disclosure, the first data represents the rotation of the first roller structure 120 as the first sensor and the first operating part of the treadmill 100 interact with each other. For example, if the first operating part is a magnetic material (or magnet), the first data are data that are obtained through the interaction based on the magnetic field between the first sensor and the first operating part.

According to an embodiment of the present disclosure, the second data represents the rotation of the second roller structure 130 as the second sensor and the second operating part of the treadmill 100 interact with each other. For example, if the second operating part is a magnetic material (or magnet), the second data are data that are obtained through the interaction based on the magnetic field between the second sensor and the second operating part.

According to various embodiments of the present disclosure, in step 930, the electronic device 300 analyzes the first data and the second data and converts the analyzed data into data related to the rotations of main wheels.

According to an embodiment of the present disclosure, the electronic device 300 analyzes the first data representing the rotation of the first roller structure 120. For example, the electronic device 300 analyzes the data related to the rotation of the first main wheel 210-1 of the main wheels of the wheelchair 200 placed on tops of the first frame 110 and the first roller structure 120, based on the first data. For example, the data related to the rotation of the first main wheel 210-1 include at least one of a rotational direction, a rotational angle, a rotational speed, a rotation time, a rotational force, and a rotational inertia of the first main wheel 210-1.

According to an embodiment of the present disclosure, the electronic device 300 analyzes the second data representing the rotation of the second roller structure 130. For example, the electronic device 300 analyzes the data related to the rotation of the second main wheel 210-2 of the main wheels of the wheelchair 200 placed on tops of the second frame 150 and the second roller structure 130, based on the second data. For example, the data related to the rotation of the second main wheel 210-2 include at least one of a rotational direction, a rotational angle, a rotational speed, a rotation time, a rotational force, and a rotational inertia of the second main wheel 210-2.

For example, the rotational angle is an angle of the corresponding main wheel that rotates from a reference angle for a predetermined time. For example, the rotation time is time during which the corresponding main wheel rotates from a time point of stop to a time point of next stop.

According to various embodiments of the present disclosure, in step 940, the electronic device 300 matches the data related to the rotation of the first main wheel 210-1 and the data related to the rotation of the second main wheel 210-2 to an input command.

According to an embodiment of the present disclosure, the electronic device 300 matches ones of the rotational directions, rotational angles, and rotational speeds of the first main wheel 210-1 and the second main wheel 210-2 to the input command.

For example, the rotational direction of the first main wheel 210-1 includes forward and backward directions, and the rotational direction of the second main wheel 210-2 includes forward and backward directions. For example, the forward direction means a direction that allows the main wheels to rotate in positive (+) directions from given angles, and the backward direction means a direction that allows the main wheels to rotate in negative (-) directions from given angles.

For example, the input command includes at least one of commands, such as select, cancel, check, continue, store, delete, refresh, search, set, reproduce, stop, pause, return to previous screen, move to next screen, return to home, character input, character input delete, move to +x-axis, move to -x-axis, move to +y-axis, and move to -y-axis.

The electronic device 300 matches the forward direction as the rotational direction of the first main wheel 210-1 and the forward direction as the rotational direction of the second main wheel 210-2 to a first input command.

The electronic device 300 matches the forward direction as the rotational direction of the first main wheel 210-1 and the backward direction as the rotational direction of the second main wheel 210-2 to a second input command.

The electronic device 300 matches the backward direction as the rotational direction of the first main wheel 210-1 and the forward direction as the rotational direction of the second main wheel 210-2 to a third input command.

The electronic device 300 matches the backward direction as the rotational direction of the first main wheel 210-1 and the backward direction as the rotational direction of the second main wheel 210-2 to a fourth input command.

The electronic device 300 matches the forward direction as the rotational direction of the first main wheel 210-1 and no rotation of the second main wheel 210-2 to a fifth input command.

According to an embodiment of the present disclosure, the fifth input command is a command that moves a cursor 1 in the first direction (e.g., in the direction of +x-axis), based on the rotational angle of the first main wheel 210-1.

For example, the electronic device 300 determines the number of times the cursor 1 moves, based on a value obtained by dividing the rotational angle of the first main wheel 210-1 into a first angle (e.g., 45°). For example, the electronic device 300 moves the cursor 1 n times in the first direction if the value obtained by dividing the rotational angle of the first main wheel 210-1 into the first angle is greater than n-1 and less than n. For example, the electronic device 300 moves the cursor 1 one time in the first direction if the value obtained by dividing the rotational angle of the first main wheel 210-1 into the first angle is greater than 0 and less than 1. For example, the electronic device 300 moves the cursor 1 two times in the first direction if the value obtained by dividing the rotational angle of the first main wheel 210-1 into the first angle is greater than 1 and less than 2. For example, the electronic device 300 moves the cursor 1 three times in the first direction if the value obtained by dividing the rotational angle of the first main wheel 210-1 into the first angle is greater than 2 and less than 3. For example, the electronic device 300 moves the cursor 1 four times in the first direction if the value obtained by dividing the rotational angle of the first main wheel 210-1 into the first angle is greater than 3 and less than 4.

According to an embodiment of the present disclosure, the fifth input command is a command that moves the cursor 1 in the first direction (e.g., in the direction of +x-axis), based on the rotational speed of the first main wheel 210-1.

For example, the electronic device 300 determines the number of times the cursor 1 moves, based on a value obtained by dividing the rotational speed of the first main wheel 210-1 into a first speed (e.g., 1 m/s). For example, the electronic device 300 moves the cursor 1 n times in the first direction if the value obtained by dividing the rotational speed of the first main wheel 210-1 into the first speed is greater than n-1 and less than n. For example, the electronic device 300 moves the cursor 1 one time in the first direction if the value obtained by dividing the rotational speed of the first main wheel 210-1 into the first speed is greater than 0 and less than 1. For example, the electronic device 300 moves the cursor 1 two times in the first direction if the value obtained by dividing the rotational speed of the first main wheel 210-1 into the first speed is greater than 1 and less than 2. For example, the electronic device 300 moves the cursor 1 three times in the first direction if the value obtained by dividing the rotational speed of the first main wheel 210-1 into the first speed is greater than 2 and less than 3. For example, the electronic device 300 moves the cursor 1 four times in the first direction if the value obtained by dividing the rotational speed of the first main wheel 210-1 into the first speed is greater than 3 and less than 4.

The electronic device 300 matches the backward direction as the rotational direction of the first main wheel 210-1 and no rotation of the second main wheel 210-2 to a sixth input command.

According to an embodiment of the present disclosure, the sixth input command is a command that moves the cursor 1 in the second direction (e.g., in the direction of -x-axis), based on the rotational angle of the first main wheel 210-1.

For example, the electronic device 300 determines the number of times the cursor 1 moves, based on a value obtained by dividing the rotational angle of the first main wheel 210-1 into a second angle (e.g., -45°). For example, the electronic device 300 moves the cursor 1 n times in the second direction if the value obtained by dividing the rotational angle of the first main wheel 210-1 into the second angle is greater than n-1 and less than n. For example, the electronic device 300 moves the cursor 1 one time in the second direction if the value obtained by dividing the rotational angle of the first main wheel 210-1 into the second angle is greater than 0 and less than 1. For example, the electronic device 300 moves the cursor 1 two times in the second direction if the value obtained by dividing the rotational angle of the first main wheel 210-1 into the second angle is greater than 1 and less than 2. For example, the electronic device 300 moves the cursor 1 three times in the first direction if the value obtained by dividing the rotational angle of the first main wheel 210-1 into the second angle is greater than 2 and less than 3. For example, the electronic device 300 moves the cursor 1 four times in the second direction if the value obtained by dividing the rotational angle of the first main wheel 210-1 into the second angle is greater than 3 and less than 4.

According to an embodiment of the present disclosure, the sixth input command is a command that moves the cursor 1 in the second direction (e.g., in the direction of -x-axis), based on the rotational speed of the first main wheel 210-1.

For example, the electronic device 300 determines the number of times the cursor 1 moves, based on a value obtained by dividing the rotational speed of the first main wheel 210-1 into a second speed (e.g., -1 m/s). For example, the electronic device 300 moves the cursor 1 n times in the second direction if the value obtained by dividing the rotational speed of the first main wheel 210-1 into the second speed is greater than n-1 and less than n. For example, the electronic device 300 moves the cursor 1 one time in the second direction if the value obtained by dividing the rotational speed of the first main wheel 210-1 into the second speed is greater than 0 and less than 1. For example, the electronic device 300 moves the cursor 1 two times in the second direction if the value obtained by dividing the rotational speed of the first main wheel 210-1 into the second speed is greater than 1 and less than 2. For example, the electronic device 300 moves the cursor 1 three times in the second direction if the value obtained by dividing the rotational speed of the first main wheel 210-1 into the second speed is greater than 2 and less than 3. For example, the electronic device 300 moves the cursor 1 four times in the second direction if the value obtained by dividing the rotational speed of the first main wheel 210-1 into the second speed is greater than 3 and less than 4.

The electronic device 300 matches no rotation of the first main wheel 210-1 and the forward direction as the rotational direction of the second main wheel 210-2 to a seventh input command.

According to an embodiment of the present disclosure, the seventh input command is a command that moves the cursor 1 in the third direction (e.g., in the direction of +y-axis), based on the rotational angle of the second main wheel 210-2.

For example, the electronic device 300 determines the number of times the cursor 1 moves, based on a value obtained by dividing the rotational angle of the second main wheel 210-2 into a first angle (e.g., 45°). For example, the electronic device 300 moves the cursor 1 n times in the third direction if the value obtained by dividing the rotational angle of the second main wheel 210-2 into the first angle is greater than n-1 and less than n. For example, the electronic device 300 moves the cursor 1 one time in the third direction if the value obtained by dividing the rotational angle of the second main wheel 210-2 into the first angle is greater than 0 and less than 1. For example, the electronic device 300 moves the cursor 1 two times in the third direction if the value obtained by dividing the rotational angle of the second main wheel 210-2 into the first angle is greater than 1 and less than 2. For example, the electronic device 300 moves the cursor 1 three times in the third direction if the value obtained by dividing the rotational angle of the second main wheel 210-2 into the first angle is greater than 2 and less than 3. For example, the electronic device 300 moves the cursor 1 four times in the third direction if the value obtained by dividing the rotational angle of the second main wheel 210-2 into the first angle is greater than 3 and less than 4.

According to an embodiment of the present disclosure, the seventh input command is a command that moves the cursor 1 in the third direction (e.g., in the direction of +y-axis), based on the rotational speed of the second main wheel 210-2.

For example, the electronic device 300 determines the number of times the cursor 1 moves, based on a value obtained by dividing the rotational speed of the second main wheel 210-2 into a first speed (e.g., 1 m/s). For example, the electronic device 300 moves the cursor 1 n times in the third direction if the value obtained by dividing the rotational speed of the second main wheel 210-2 into the first speed is greater than n-1 and less than n. For example, the electronic device 300 moves the cursor 1 one time in the third direction if the value obtained by dividing the rotational speed of the second main wheel 210-2 into the first speed is greater than 0 and less than 1. For example, the electronic device 300 moves the cursor 1 two times in the third direction if the value obtained by dividing the rotational speed of the second main wheel 210-2 into the first speed is greater than 1 and less than 2. For example, the electronic device 300 moves the cursor 1 three times in the third direction if the value obtained by dividing the rotational speed of the second main wheel 210-2 into the first speed is greater than 2 and less than 3. For example, the electronic device 300 moves the cursor 1 four times in the third direction if the value obtained by dividing the rotational speed of the second main wheel 210-2 into the first speed is greater than 3 and less than 4.

The electronic device 300 matches no rotation of the first main wheel 210-1 and the backward direction as the rotational direction of the second main wheel 210-2 to an eighth input command.

According to an embodiment of the present disclosure, the eighth input command is a command that moves the cursor 1 in the fourth direction (e.g., in the direction of -y-axis), based on the rotational angle of the second main wheel 210-2.

For example, the electronic device 300 determines the number of times the cursor 1 moves, based on a value obtained by dividing the rotational angle of the second main wheel 210-2 into a second angle (e.g., -45°). For example, the electronic device 300 moves the cursor 1 n times in the fourth direction if the value obtained by dividing the rotational angle of the second main wheel 210-2 into the second angle is greater than n-1 and less than n. For example, the electronic device 300 moves the cursor 1 one time in the fourth direction if the value obtained by dividing the rotational angle of the second main wheel 210-2 into the second angle is greater than 0 and less than 1. For example, the electronic device 300 moves the cursor 1 two times in the fourth direction if the value obtained by dividing the rotational angle of the second main wheel 210-2 into the second angle is greater than 1 and less than 2. For example, the electronic device 300 moves the cursor 1 three times in the fourth direction if the value obtained by dividing the rotational angle of the second main wheel 210-2 into the second angle is greater than 2 and less than 3. For example, the electronic device 300 moves the cursor 1 four times in the fourth direction if the value obtained by dividing the rotational angle of the second main wheel 210-2 into the second angle is greater than 3 and less than 4.

According to the embodiment of the present disclosure, the eighth input command is a command that moves the cursor 1 in the fourth direction (e.g., in the direction of -y-axis), based on the rotational speed of the second main wheel 210-2.

For example, the electronic device 300 determines the number of times the cursor 1 moves, based on a value obtained by dividing the rotational speed of the second main wheel 210-2 into a second speed (e.g., -1 m/s). For example, the electronic device 300 moves the cursor 1 n times in the fourth direction if the value obtained by dividing the rotational speed of the second main wheel 210-2 into the second speed is greater than n-1 and less than n. For example, the electronic device 300 moves the cursor 1 one time in the fourth direction if the value obtained by dividing the rotational speed of the second main wheel 210-2 into the second speed is greater than 0 and less than 1. For example, the electronic device 300 moves the cursor 1 two times in the fourth direction if the value obtained by dividing the rotational speed of the second main wheel 210-2 into the second speed is greater than 1 and less than 2. For example, the electronic device 300 moves the cursor 1 three times in the fourth direction if the value obtained by dividing the rotational speed of the second main wheel 210-2 into the second speed is greater than 2 and less than 3. For example, the electronic device 300 moves the cursor 1 four times in the fourth direction if the value obtained by dividing the rotational speed of the second main wheel 210-2 into the second speed is greater than 3 and less than 4.

According to various embodiments of the present disclosure, in step 950, the electronic device 300 controls the application according to the input command.

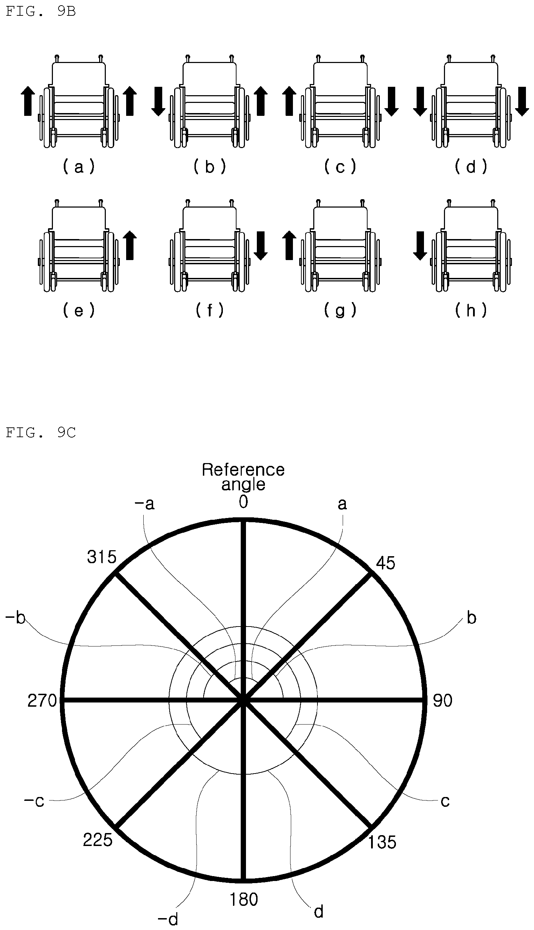

FIG. 9B is a schematic view showing an example of operations related to the rotations of the main wheels according to the exemplary embodiment of the present disclosure.

According to various embodiments of the present disclosure, in the step 930 of FIG. 9A, the electronic device 300 analyzes the first data and the second data and converts the analyzed data into the data related to the rotations of the main wheels. For example, the data related to the rotations of the main wheels include ones of rotational directions, rotational angles, rotational speeds, rotation time, rotational forces, and rotational inertias of the main wheels.

According to an embodiment of the present disclosure, the electronic device 300 matches the rotational directions of the first main wheel 210-1 and the second main wheel 210-2 to an input command. For example, the rotational direction of the first main wheel 210-1 includes forward and backward directions, and the rotational direction of the second main wheel 210-2 includes forward and backward directions.

As shown in a front view (a) of the wheelchair 200, the electronic device 300 matches the forward direction as the rotational direction of the first main wheel 210-1 and the forward direction as the rotational direction of the second main wheel 210-2 to the first input command.

As shown in a front view (b) of the wheelchair 200, the electronic device 300 matches the forward direction as the rotational direction of the first main wheel 210-1 and the backward direction as the rotational direction of the second main wheel 210-2 to the second input command.

As shown in a front view (c) of the wheelchair 200, the electronic device 300 matches the backward direction as the rotational direction of the first main wheel 210-1 and the forward direction as the rotational direction of the second main wheel 210-2 to the third input command.

As shown in a front view (d) of the wheelchair 200, the electronic device 300 matches the backward direction as the rotational direction of the first main wheel 210-1 and the backward direction as the rotational direction of the second main wheel 210-2 to the fourth input command.

As shown in a front view (e) of the wheelchair 200, the electronic device 300 matches the forward direction as the rotational direction of the first main wheel 210-1 and no rotation of the second main wheel 210-2 to the fifth input command.

As shown in a front view (f) of the wheelchair 200, the electronic device 300 matches the backward direction as the rotational direction of the first main wheel 210-1 and no rotation of the second main wheel 210-2 to the sixth input command.

As shown in a front view (g) of the wheelchair 200, the electronic device 300 matches no rotation of the first main wheel 210-1 and the forward direction as the rotational direction of the second main wheel 210-2 to the seventh input command.

As shown in a front view (h) of the wheelchair 200, the electronic device 300 matches no rotation of the first main wheel 210-1 and the backward direction as the rotational direction of the second main wheel 210-2 to the eighth input command.

FIG. 9C is a schematic view showing an example related to rotational angles of the main wheels according to the exemplary embodiment of the present disclosure.

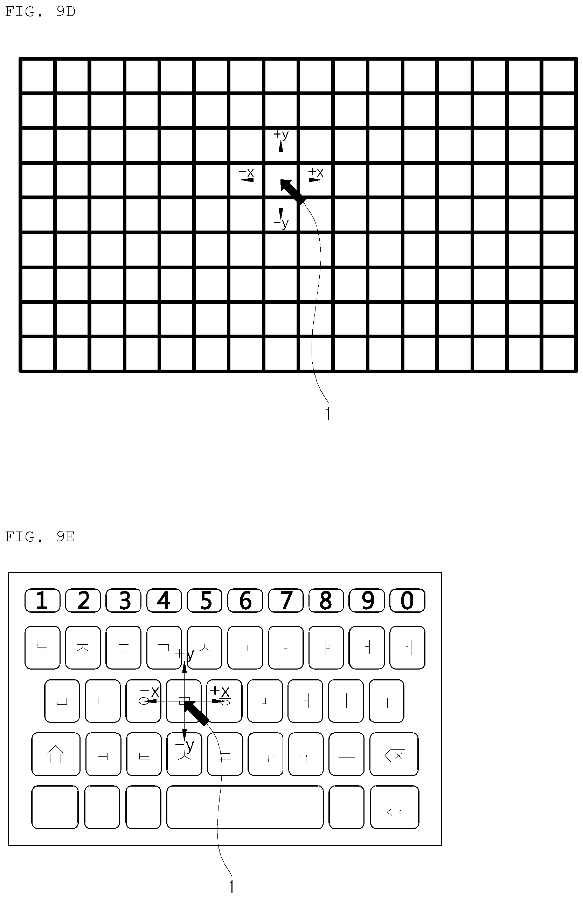

FIGs. 9D to 9F are schematic views showing examples of operations of the electronic device for controlling the application according to matched input commands according to the exemplary embodiment of the present disclosure.

According to an embodiment of the present disclosure, the electronic device 300 matches the rotational directions of the first main wheel 210-1 and the second main wheel 210-2 to the input command. The electronic device 300 moves the cursor 1 in the predetermined direction (e.g., in the direction of +x, -x, +y, or -y-axis), based on the rotational angle of the first main wheel 210-1 and/or the rotational angle of the second main wheel 210-2 according to at least one of the fifth input command, the sixth input command, the seventh input command, and the eighth input command.

For example, the fifth input command is a command that moves the cursor 1 in the first direction (in the direction of +x-axis), based on the rotational angle of the first main wheel 210-1, and the sixth input command is a command that moves the cursor 1 in the second direction (in the direction of -x-axis), based on the rotational angle of the first main wheel 210-1. The seventh input command is a command that moves the cursor 1 in the third direction (in the direction of +y-axis), based on the rotational angle of the second main wheel 210-2, and the eighth input command is a command that moves the cursor 1 in the fourth direction (in the direction of -y-axis), based on the rotational angle of the second main wheel 210-2.

According to an embodiment of the present disclosure, the electronic device 300 matches the forward rotation of the first main wheel 210-1 to the fifth input command and thus moves the cursor 1. For example, if the rotational angle of the first main wheel 210-1 is a and a value obtained by dividing the angle a into the first angle (e.g., 45°) is greater than 0 and less than 1, the electronic device 300 moves the cursor 1 one time in the first direction (in the direction of +x-axis). For example, if the rotational angle of the first main wheel 210-1 is b and a value obtained by dividing the angle b into the first angle is greater than 1 and less than 2, the electronic device 300 moves the cursor 1 two times in the first direction. For example, if the rotational angle of the first main wheel 210-1 is c and a value obtained by dividing the angle c into the first angle is greater than 2 and less than 3, the electronic device 300 moves the cursor 1 three times in the first direction. For example, if the rotational angle of the first main wheel 210-1 is d and a value obtained by dividing the angle d into the first angle is greater than 3 and less than 4, the electronic device 300 moves the cursor 1 four times in the first direction.

According to an embodiment of the present disclosure, the electronic device 300 matches the backward rotation of the first main wheel 210-1 to the sixth input command and thus moves the cursor 1. For example, if the rotational angle of the first main wheel 210-1 is -a and a value obtained by dividing the angle -a into the second angle (e.g., -45°) is greater than 0 and less than 1, the electronic device 300 moves the cursor 1 one time in the second direction (in the direction of -x-axis). For example, if the rotational angle of the first main wheel 210-1 is -b and a value obtained by dividing the angle -b into the second angle is greater than 1 and less than 2, the electronic device 300 moves the cursor 1 two times in the second direction. For example, if the rotational angle of the first main wheel 210-1 is -c and a value obtained by dividing the angle -c into the second angle is greater than 2 and less than 3, the electronic device 300 moves the cursor 1 three times in the second direction. For example, if the rotational angle of the first main wheel 210-1 is -d and a value obtained by dividing the angle -d into the second angle is greater than 3 and less than 4, the electronic device 300 moves the cursor 1 four times in the second direction.

According to an embodiment of the present disclosure, the electronic device 300 matches the forward rotation of the second main wheel 210-2 to the seventh input command and thus moves the cursor 1. For example, if the rotational angle of the second main wheel 210-2 is a and a value obtained by dividing the angle a into the first angle (e.g., 45°) is greater than 0 and less than 1, the electronic device 300 moves the cursor 1 one time in the third direction (in the direction of +y-axis). For example, if the rotational angle of the second main wheel 210-2 is b and a value obtained by dividing the angle b into the first angle is greater than 1 and less than 2, the electronic device 300 moves the cursor 1 two times in the third direction. For example, if the rotational angle of the second main wheel 210-2 is c and a value obtained by dividing the angle c into the first angle is greater than 2 and less than 3, the electronic device 300 moves the cursor 1 three times in the third direction. For example, if the rotational angle of the second main wheel 210-2 is d and a value obtained by dividing the angle d into the first angle is greater than 3 and less than 4, the electronic device 300 moves the cursor 1 four times in the third direction.

According to an embodiment of the present disclosure, the electronic device 300 matches the backward rotation of the second main wheel 210-2 to the eighth input command and thus moves the cursor 1. For example, if the rotational angle of the second main wheel 210-2 is -a and a value obtained by dividing the angle -a into the second angle (e.g., -45°) is greater than 0 and less than 1, the electronic device 300 moves the cursor 1 one time in the fourth direction (in the direction of -y-axis). For example, if the rotational angle of the second main wheel 210-2 is -b and a value obtained by dividing the angle -b into the second angle is greater than 1 and less than 2, the electronic device 300 moves the cursor 1 two times in the fourth direction. For example, if the rotational angle of the second main wheel 210-2 is -c and a value obtained by dividing the angle -c into the second angle is greater than 2 and less than 3, the electronic device 300 moves the cursor 1 three times in the fourth direction. For example, if the rotational angle of the second main wheel 210-2 is -d and a value obtained by dividing the angle -d into the second angle is greater than 3 and less than 4, the electronic device 300 moves the cursor 1 four times in the fourth direction.

FIG. 9D is a schematic view showing the example of operations of the electronic device 300 for moving the cursor 1 on the screen according to the input command in the exemplary embodiment of the present disclosure.

According to various embodiments of the present disclosure, the electronic device 300 moves the cursor 1 in the predetermined direction (e.g., in the direction of +x, -x, +y, or -y-axis), based on the rotational angle of the first main wheel 210-1 and/or the rotational angle of the second main wheel 210-2, according to at least one of the fifth input command, the sixth input command, the seventh input command, and the eighth input command.

According to various embodiments of the present disclosure, the electronic device 300 is based on the input command selected (e.g., the first input command) at the position to which the cursor 1 moves and thus selects the corresponding region in the application.

FIG. 9E is a schematic view showing the example of operations of the electronic device 300 for inputting characters on a keyboard according to the input command in the exemplary embodiment of the present disclosure.

According to various embodiments of the present disclosure, the electronic device 300 moves the cursor 1 on the keyboard in the predetermined direction (e.g., in the direction of +x, -x, +y, or -y-axis), based on the rotational angle of the first main wheel 210-1 and/or the rotational angle of the second main wheel 210-2, according to at least one of the fifth input command, the sixth input command, the seventh input command, and the eighth input command.

According to various embodiments of the present disclosure, the electronic device 300 is based on the input command selected (e.g., the first input command) at the position to which the cursor 1 moves and thus selects the corresponding character on the keyboard.

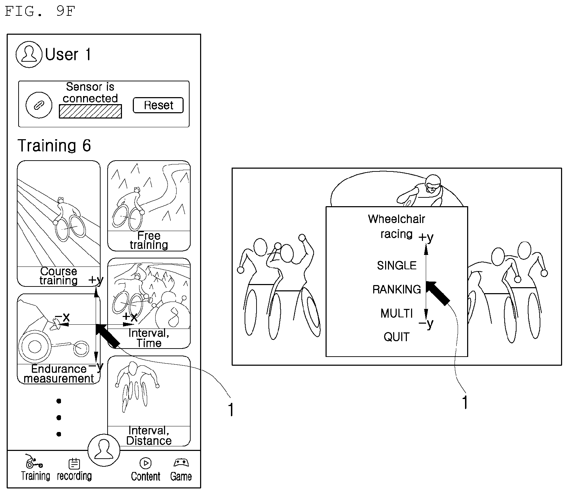

FIG. 9F is a schematic view showing the example of operations of the electronic device 300 for moving the cursor 1 on a menu selection screen according to the input command in the exemplary embodiment of the present disclosure.

According to various embodiments of the present disclosure, the electronic device 300 moves the cursor 1 on the menu selection screen in the predetermined direction (e.g., in the direction of +x, -x, +y, or -y-axis), based on the rotational angle of the first main wheel 210-1 and/or the rotational angle of the second main wheel 210-2, according to at least one of the fifth input command, the sixth input command, the seventh input command, and the eighth input command.

According to various embodiments of the present disclosure, the electronic device 300 is based on the input command selected (e.g., the first input command) at the position to which the cursor 1 moves and thus selects the corresponding menu in the application.

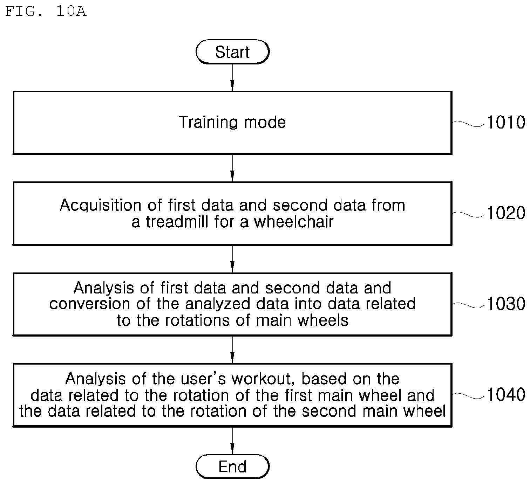

FIG. 10A is a schematic flowchart showing an example of operations of the electronic device according to the exemplary embodiment of the present disclosure.

According to various embodiments of the present disclosure, in step 1010, the electronic device 300 executes a training mode.

According to an embodiment of the present disclosure, the electronic device 300 converts the application into a training mode, based on the user's input. For example, the user's input includes at least one of the first input command, the second input command, the third input command, the fourth input command, the fifth input command, the sixth input command, the seventh input command, and the eighth input command based on at least one of the rotational direction, the rotational angle, and the rotational speed of the first main wheel 210-1 and/or the second main wheel 210-2.

For example, the training mode is a program for the user's wheelchair training. The training mode is a mode that trains the user with a given activity, such as pulling both sides, pushing both sides, twist, pushing left side, and pushing right side of the wheelchair 200, or a mode that trains the user with the combination of at least one or more activities, such as balance-keeping training, speed-adjusting training, hurdle-overcoming training, and long distance training, to improve his or her workout ability.

According to various embodiments of the present disclosure, in step 1020, the electronic device 300 acquires the first data and second data from the treadmill 100 for the wheelchair 200 through the communication module.

The electronic device 300 performs the step 1020 in the same manner as the step 920 of FIG. 9A.

According to various embodiments of the present disclosure, in step 1030, the electronic device 300 analyzes the first data and the second data and converts the analyzed data into data related to the rotations of the main wheels.

The electronic device 300 performs the step 1030 in the same manner as the step 930 of FIG. 9A.

According to various embodiments of the present disclosure, in step 1040, the electronic device 300 analyzes the user's workout, based on the data related to the rotation of the first main wheel 210-1 and the data related to the rotation of the second main wheel 210-2.

According to an embodiment of the present disclosure, the electronic device 300 analyzes at least one of a rotational direction, a rotational angle, a rotational speed, a rotation time, a rotational force, and a rotational inertia of the first main wheel 210-1 and/or the second main wheel 210-2.

According to an embodiment of the present disclosure, the electronic device 300 analyzes at least one of time during which the user completes a given activity, an average rotational speed while the given activity is being performed, a change in rotational speeds while the given activity is being performed, a change in rotational forces while the given activity is being performed, a rotational inertia when the given activity is started or stopped, and a degree of correspondence of the given activity to a training activity, based on the data related to the rotation of the first main wheel 210-1 and/or the second main wheel 210-2.

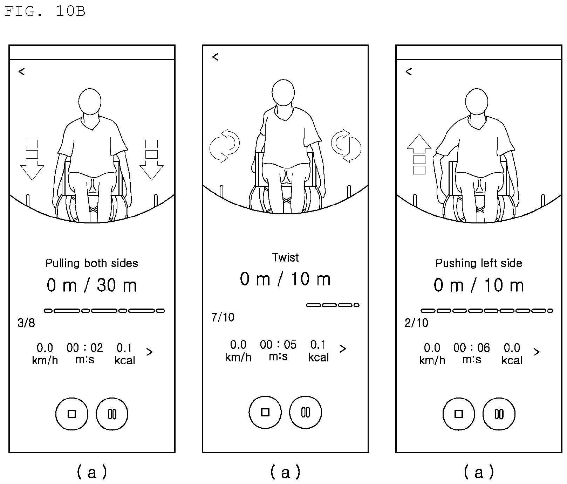

FIG. 10B is a schematic view showing an example of the training mode of the electronic device according to the exemplary embodiment of the present disclosure.

A front view (a) of FIG. 10B shows the user's training for pulling both sides. Through the training for pulling both sides, for example, the electronic device 300 trains the user in improving the muscular force of his or her upper body, the technology in manipulating the wheelchair 200, and his or her endurance.

A front view (b) of FIG. 10B shows twist training. Through the twist training, for example, the electronic device 300 trains the user in improving the muscular force of his or her core, his or her body flexibility, his or her physical strength, and his or her endurance.

A front view (c) of FIG. 10B shows the user's training for pushing the left side. Through the training for pushing the left side, for example, the electronic device 300 trains the user in improving his or her technology in rotating the wheelchair, a specific muscular force of his or her upper body, his or her physical strength, and his or her endurance.

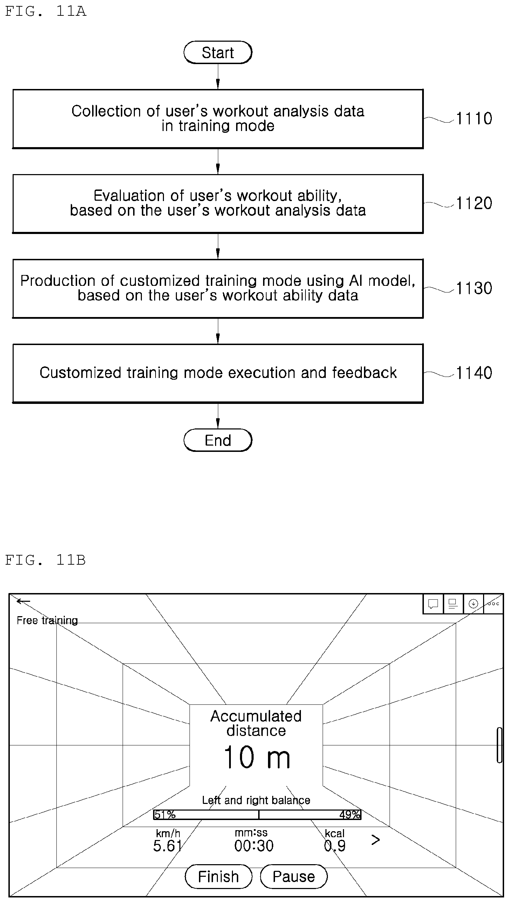

FIG. 11A is a schematic flowchart showing another example of operations of the electronic device according to the exemplary embodiment of the present disclosure.

According to various embodiments of the present disclosure, in step 1110, the electronic device 300 collects the user's workout analysis data in the training mode.

For example, the electronic device 300 analyzes at least one of the user's workout analysis data selected from time during which the user completes the given activity, an average rotational speed while the given activity is being performed, a change in rotational speeds while the given activity is being performed, a change in rotational forces while the given activity is being performed, a rotational inertia when the given activity is started or stopped, and a degree of correspondence of the given activity to a training activity, which have been analyzed in the step 1040 of FIG. 10A.

According to various embodiments of the present disclosure, in step 1120, the electronic device 300 evaluates the user's workout ability, based on the user's workout analysis data.

For example, the electronic device 300 evaluates the user's control ability to precisely control the rotational direction, the rotational angle, and the rotational speed of the wheelchair. For example, the electronic device 300 evaluates the muscular force of the user's upper body that is required in pushing or pulling the wheelchair 200, based on the data of the rotational force. For example, the electronic device 300 evaluates the user's reaction speed capable of rapidly reacting with the rotation of the wheelchair 200 and controlling the wheelchair, based on the rotational speed and time. For example, the electronic device 300 evaluates the user's endurance capable of performing the continuous workouts and controlling the wheelchair 200 for long hours through long distance wheelchair training. For example, the electronic device 300 evaluates the user's skill capable of accurately controlling the wheelchair 200, based on the data of the rotational angle. For example, the electronic device 300 evaluates the user's mobility capable of freely moving the wheelchair 200 in various environments.

According to various embodiments of the present disclosure, in step 1130, the electronic device 300 produces a customized training mode using an AI model, based on the user's workout ability data.

According to an embodiment of the present disclosure, the electronic device 300 inputs the user's workout ability data evaluated in the step 1120 to the AI model. For example, the electronic device 300 inputs at least one of the user's control ability, the muscular force of the user's upper body, the user's reaction speed, the user's endurance, the user's skill degree, and the user's mobility to the AI model.

For example, the AI model is an artificial intelligence model that is trained with the user's workout ability data such as the user's control ability, the muscular force of the user's upper body, the user's reaction speed, the user's endurance, the user's skill degree, and the user's mobility, and if the user's workout ability data is inputted to the AI model, the AI model outputs a customized training mode. The AI model is an artificial neural network model that is built with a given language and has a plurality of layers and/or operations. The AI model is one of various types of networks such as convolution neural network (CNN), region with convolution neural network (R-CNN), region proposal network (RPN), recurrent neural network (RNN), stacking-based deep neural network (S-DNN), state-space dynamic neural network (S-SDNN), deconvolution network, deep belief network (DBN), restricted Boltzmann machine (RBM), fully convolutional network, long short-term memory (LSTM) network, and classification network.

According to an embodiment of the present disclosure, the electronic device 300 produces the customized training mode from the AI model.

The electronic device 300 produces the customized training mode according to the user's workout ability.

If the value of the user's workout ability data is less than an average data value, the customized training mode is a mode that trains the user in improving his or her workout ability with the lower data value than the average data value. For example, the electronic device 300 produces the customized training mode for training a given activity repeatedly so that the workout ability for the given activity can be improved.

For example, if the value of the user's workout ability data is greater than or equal to the average data value, the customized training mode is a mode that trains the user in increasing a level of difficulty in his or her workout to strengthen the user's overall workout ability, based on the history of the user's workout ability when he or she executes the training mode. For example, the electronic device 300 produces the customized training mode for training various activities alternately so that a training intensity in the current training mode is stronger than that in the past training mode to improve his or her overall workout ability.

According to various embodiments of the present disclosure, in step 1140, the electronic device 300 executes the customized training mode and gives the feedback of the customized training mode.

According to an embodiment of the present disclosure, the electronic device 300 executes the customized training mode produced in the step 1130, collects the user's workout analysis data in the customized training mode, and evaluates the user's workout ability.

According to an embodiment of the present disclosure, the electronic device 300 updates the AI model, based on the user's workout ability data evaluated in the customized training mode. For example, the electronic device 300 corrects the weight value of the AI model, based on the user's workout ability data.

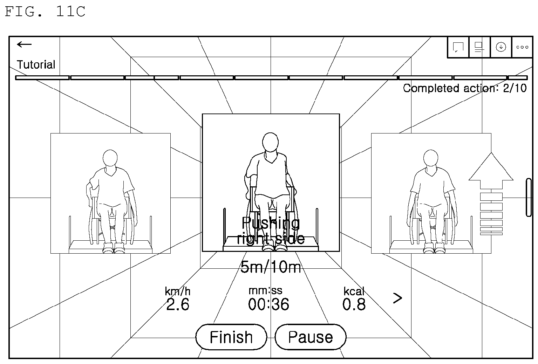

FIG. 11B is a schematic view showing an example of a training mode result according to the exemplary embodiment of the present disclosure, and FIG. 11C is a schematic view showing an example of the customized training mode according to the exemplary embodiment of the present disclosure.

Referring to FIG. 11B, in the case of the left and right balance of the user in the training mode, his or her left side balance is 510, whereas his or her right side balance is 490, and therefore, the muscular force in his or her right upper body is lower than that in his or her left upper body.

According to an embodiment of the present disclosure, the electronic device 300 inputs the muscular force data of the left and right upper body in the training mode to the AI model and thus produces a customized training mode for balancing the user's left and right sides.

FIG. 11C shows the example of the customized training mode produced from the AI model, in which an activity, 'pushing the right side' is trained to improve the user's poor muscular force in his or her right upper body.

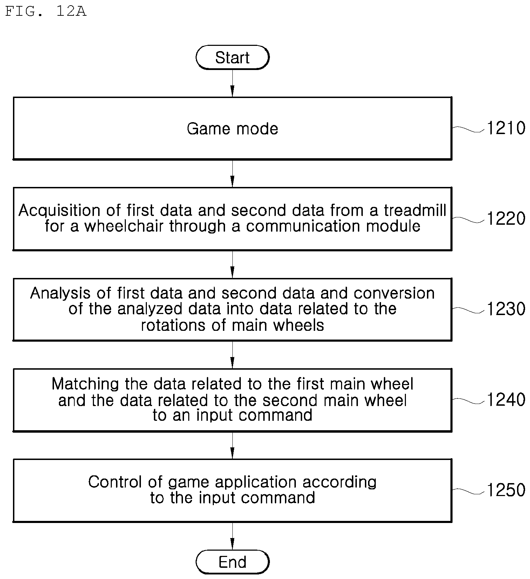

FIG. 12A is a schematic flowchart showing an example of operations of the electronic device according to the exemplary embodiment of the present disclosure.

According to various embodiments of the present disclosure, in step 1210, the electronic device 300 executes a game mode.

According to an embodiment of the present disclosure, the electronic device 300 provides game application images and characters to the display so that a game is played through the user's operations performed with the wheelchair 200.

According to an embodiment of the present disclosure, the electronic device 300 converts the application into a game mode, based on the user's input. For example, the user's input includes at least one of the first input command, the second input command, the third input command, the fourth input command, the fifth input command, the sixth input command, the seventh input command, and the eighth input command based on the operation of the first main wheel 210-1 and/or the second main wheel 210-2.

For example, the game mode is a game program that executes missions through the operations of the wheelchair 200.

According to various embodiments of the present disclosure, in step 1220, the electronic device 300 acquires the first data and second data from the treadmill 100 for the wheelchair 200 through the communication module.

The electronic device 300 performs the step 1220 in the same manner as the step 920 of FIG. 9A.

According to various embodiments of the present disclosure, in step 1230, the electronic device 300 analyzes the first data and the second data and converts the analyzed data into data related to the rotations of the main wheels.

The electronic device 300 performs the step 1230 in the same manner as the step 930 of FIG. 9A.

According to various embodiments of the present disclosure, in step 1240, the electronic device 300 matches the data related to the rotation of the first main wheel 210-1 and the data related to the rotation of the second main wheel 210-2 to an input command.

According to an embodiment of the present disclosure, the input command in a running game includes at least one of a forward movement command, a backward movement command, a forward left turn command, a forward right turn command, a backward left turn command, a backward right turn command, a movement command to +z-axis, and movement command to -z-axis of a game character. For example, the electronic device 300 matches the forward direction as the rotational direction of the first main wheel 210-1 and the forward direction as the rotational direction of the second main wheel 210-2 to the forward movement command of the game character. The electronic device 300 matches the forward direction as the rotational direction of the first main wheel 210-1 and the backward direction as the rotational direction of the second main wheel 210-2 to the movement command to the +z-axis of the game character. The electronic device 300 matches the backward direction as the rotational direction of the first main wheel 210-1 and the forward direction as the rotational direction of the second main wheel 210-2 to the movement command to the -z-axis of the game character. The electronic device 300 matches the backward direction as the rotational direction of the first main wheel 210-1 and the backward direction as the rotational direction of the second main wheel 210-2 to the backward movement command of the game character. The electronic device 300 matches the forward direction as the rotational direction of the first main wheel 210-1 and no rotation of the second main wheel 210-2 to the forward right turn command of the game character. The electronic device 300 matches the backward direction as the rotational direction of the first main wheel 210-1 and no rotation of the second main wheel 210-2 to the backward right turn command of the game character. The electronic device 300 matches no rotation of the first main wheel 210-1 and the forward rotation as the rotational direction of the second main wheel 210-2 to the forward left turn command of the game character. The electronic device 300 matches no rotation of the first main wheel 210-1 and the backward rotation as the rotational direction of the second main wheel 210-2 to the backward left turn command of the game character. For example, the electronic device 300 determines a moving speed of the game character, based on the rotational speed of the first main wheel 210-1 and/or the rotational speed of the second main wheel 210-2.

According to an embodiment of the present disclosure, the input command in a platform game includes at least one of a movement command to +x-axis, a movement command to -x-axis, a movement command to +y-axis, a movement commend to -y-axis, a jump command, a sneaking command, an attack command, and a dodge command of a game character.

For example, the electronic device 300 matches the forward direction as the rotational direction of the first main wheel 210-1 and the forward direction as the rotational direction of the second main wheel 210-2 to the attack command of the game character. The electronic device 300 matches the forward direction as the rotational direction of the first main wheel 210-1 and the backward direction as the rotational direction of the second main wheel 210-2 to the jump command of the game character. The electronic device 300 matches the backward direction as the rotational direction of the first main wheel 210-1 and the forward direction as the rotational direction of the second main wheel 210-2 to the sneaking command of the game character. The electronic device 300 matches the backward direction as the rotational direction of the first main wheel 210-1 and the backward direction as the rotational direction of the second main wheel 210-2 to the dodge command of the game character. The electronic device 300 matches the forward direction as the rotational direction of the first main wheel 210-1 and no rotation of the second main wheel 210-2 to the movement command to the +x-axis of the game character. The electronic device 300 matches the backward direction as the rotational direction of the first main wheel 210-1 and no rotation of the second main wheel 210-2 to the movement command to the -x-axis of the game character. The electronic device 300 matches no rotation of the first main wheel 210-1 and the forward rotation as the rotational direction of the second main wheel 210-2 to the movement command to the +y-axis of the game character. The electronic device 300 matches no rotation of the first main wheel 210-1 and the backward rotation as the rotational direction of the second main wheel 210-2 to the movement command to the -y-axis of the game character. For example, the electronic device 300 determines a moving speed of the game character, based on the rotational speed of the first main wheel 210-1 and/or the rotational speed of the second main wheel 210-2.

According to various embodiments of the present disclosure, in step 1250, the electronic device 300 controls the game application according to the input command.

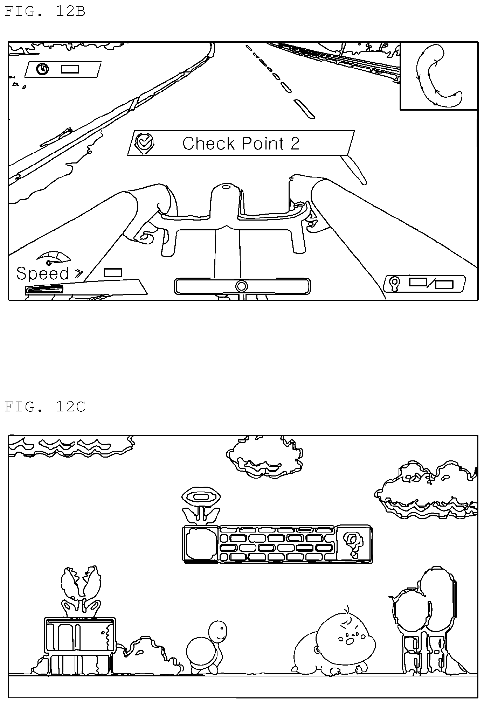

FIG. 12B is a schematic view showing an example of a game application screen provided by the electronic device according to the exemplary embodiment of the present disclosure.

According to an embodiment of the present disclosure, the electronic device 300 provides game application images and characters to the display so that a game is played through the user's operations performed with the wheelchair 200.

For example, the game application is a running game. The user operates the first main wheel 210-1 and/or the second main wheel 210-2 to control the game character so that the game character moves forward, moves backward, moves forward and turns left, moves forward and turns right, moves backward and turns left, moves backward and turns right, moves to the +z-axis, or moves to the -z-axis.

FIG. 12C is a schematic view showing another example of the game application screen provided by the electronic device according to the exemplary embodiment of the present disclosure.

According to an embodiment of the present disclosure, the electronic device 300 provides game application images and characters to the display so that a game is played through the user's operations performed with the wheelchair 200.

For example, the game application is a platform game. The user operates the first main wheel 210-1 and/or the second main wheel 210-2 to control the game character so that the game character moves to the +x-axis, moves to the -x-axis, moves to the +y-axis, moves to the -y-axis, jumps, sneaks, attacks, or dodges.

Further, the embodiment wherein the information of the user's workout and/or the information of guiding the user's workout is provided through the electronic device 300 has been explained, but the present disclosure may not be limited thereto. For example, the information of the user's workout and/or the information of guiding the user's workout may be provided through the treadmill 100 for the wheelchair 200.

As described above, the electronic device according to the exemplary embodiment of the present disclosure, can acquire the information of the workout of the user using the treadmill for the wheelchair, and therefore, the electronic device, which communicates with the treadmill for the wheelchair, can provide various types of content capable of giving motivation for the user.

While the present invention has been described with reference to the particular illustrative embodiments, it is not to be restricted by the embodiments but only by the appended claims. It is to be appreciated that those skilled in the art can change or modify the embodiments without departing from the scope and spirit of the present invention. It is therefore intended that the scope of the invention be limited not by this detailed description, but rather by the claims appended hereto.

**Claims**

1. An electronic device comprising:

   a communication module communicating with a treadmill for a wheelchair;
   a display for outputting a screen of an application; and
   a processor,
   wherein in an input mode, the processor acquires first data representing the rotation of a first roller structure of the treadmill for the wheelchair and second data representing the rotation of a second roller structure of the treadmill for the wheelchair from the treadmill for the wheelchair through the communication module, analyzes the first data and the second data to convert the analyzed data into data related to the rotation of a first main wheel and data related to the rotation of a second main wheel of the wheelchair placed on top of the treadmill for the wheelchair, matches the data related to the rotation of the first main wheel and the data related to the rotation of the second main wheel to an input command, and controls the application according to the input command.

2. The electronic device according to claim 1, wherein the data related to the rotation of the first main wheel comprise a rotational direction of the first main wheel, the data related to the rotation of the second main wheel comprise a rotational direction of the second main wheel, and the processor matches a forward direction as the rotational direction of the first main wheel and a forward direction as the rotational direction of the second main wheel to a first input command, matches the forward direction as the rotational direction of the first main wheel and a backward direction as the rotational direction of the second main wheel to a second input command, matches a backward direction as the rotational direction of the first main wheel and the forward direction as the rotational direction of the second main wheel to a third input command, matches the backward direction as the rotational direction of the first main wheel and the backward direction as the rotational direction of the second main wheel to a fourth input command, matches the forward direction as the rotational direction of the first main wheel and no rotation of the second main wheel to a fifth input command, matches the backward direction as the rotational direction of the first main wheel and no rotation of the second main wheel to a sixth input command, matches no rotation of the first main wheel and the forward direction as the rotational direction of the second main wheel to a seventh input command, and matches no rotation of the first main wheel and the backward direction as the rotational direction of the second main wheel to an eighth input command.

3. The electronic device according to claim 2, wherein the data related to the rotation of the first main wheel comprise a rotational angle of the first main wheel, the data related to the rotation of the second main wheel comprise a rotational angle of the second main wheel, the fifth input command is a command that moves a cursor in a first direction, based on the rotational angle of the first main wheel, the sixth input command is a command that moves the cursor in a second direction, based on the rotational angle of the first main wheel, the seventh input command is a command that moves the cursor in a third direction, based on the rotational angle of the second main wheel, and the eighth input command is a command that moves the cursor in a fourth direction, based on the rotational angle of the second main wheel.

4. The electronic device according to claim 3, wherein the processor executes a training mode according to the input command, acquires the first data and the second data from the treadmill for the wheelchair through the communication module, analyzes the first data and the second data to convert the analyzed data into the data related to the rotation of the first main wheel and the data related to the rotation of the second main wheel, and analyzes a user's workout,

EP 4 560 441 A1

based on the data related to the rotation of the first main wheel and the data related to the rotation of the second main wheel.

5. The electronic device according to claim 4, wherein the processor collects the user's workout analysis data in the training mode, evaluates the user's workout ability, based on the user's workout analysis data, and produces a customized training mode using an artificial intelligence model, based on the user's workout ability data evaluated.

6. The electronic device according to claim 5, wherein if the value of the user's workout ability data for a given workout is less than an average data value, the processor produces the customized training mode for training the user in strengthening his or her workout ability with the data value less than the average data value.

7. The electronic device according to claim 5, wherein if the value of the user's workout ability data is greater than or equal to the average data value, the processor produces the customized training mode for training the user in strengthening the user's overall workout ability, based on the history of the user's workout ability data.

8. The electronic device according to claim 5, wherein the processor executes the produced customized training mode, collects the user's workout analysis data in the customized training mode to evaluate the user's workout ability, and updates the artificial intelligence model, based on the user's workout analysis data evaluated in the customized training mode.

9. The electronic device according to claim 3, wherein the processor executes a game mode according to the input command, acquires the first data and the second data from the treadmill for the wheelchair through the communication module, analyzes the first data and the second data to convert the analyzed data into the data related to the rotation of the first main wheel and the data related to the rotation of the second main wheel, matches the data related to the rotation of the first main wheel and the data related to the rotation of the second main wheel to the input command, and controls a game character in the game mode according to the input command.

10. The electronic device according to claim 9, wherein if the game mode is a running game, the processor matches a forward direction as the rotational direction of the first main wheel and a forward direction as the rotational direction of the second main wheel to a forward movement command of the game character, matches the forward direction as the rotational direction of the first main wheel and a backward direction as the rotational direction of the second main wheel to a movement command to a +z-axis of the game character, matches a backward direction as the rotational direction of the first main wheel and the forward direction as the rotational direction of the second main wheel to a movement command to a -z-axis of the game character, matches the backward direction as the rotational direction of the first main wheel and the backward direction as the rotational direction of the second main wheel to a backward movement command of the game character, matches the forward direction as the rotational direction of the first main wheel and no rotation of the second main wheel to a forward right turn command of the game character, matches the backward direction as the rotational direction of the first main wheel and no rotation of the second main wheel to a backward right turn command of the game character, matches no rotation of the first main wheel and the forward rotation as the rotational direction of the second main wheel to a forward left turn command of the game character, and matches no rotation of the first main wheel and the backward rotation as the rotational direction of the second main wheel to a backward left turn command of the game character, the processor determining a moving speed of the game character, based on a rotational speed of the first main wheel and/or a rotational speed of the second main wheel.

FIG. 1

FIG. 2

FIG. 3

FIG. 4

100

FIG. 5

FIG. 6

FIG. 7

161
162 } 160
163
164

171 } 170
172

FIG. 8

FIG. 9A

Start

| Conversion to input mode | 910 |

| Acquisition of first data and second data from a treadmill through a communication module | 920 |

| Analysis of first data and second data and conversion of the analyzed data into data related to the rotations of main wheels | 930 |

| Matching the data related to the rotation of the first main wheel and the data related to the rotation of the second main wheel to an input command | 940 |

| Control of application according to the input command | 950 |

End

FIG. 9B

( a )        ( b )        ( c )        ( d )

( e )        ( f )        ( g )        ( h )

FIG. 9C

Reference
angle
0

-a      a

315      45

-b      b

270      90

-c      c

225      135

-d    180    d

FIG. 9D

1

FIG. 9E

1

FIG. 9F

FIG. 10A

```
            ( Start )
                │
                ▼
┌─────────────────────────────────────┐
│                                     │
│           Training mode             │ ～1010
│                                     │
└─────────────────────────────────────┘
                │
                ▼
┌─────────────────────────────────────┐
│  Acquisition of first data and second data from │
│       a treadmill for a wheelchair   │ ～1020
│                                     │
└─────────────────────────────────────┘
                │
                ▼
┌─────────────────────────────────────┐
│    Analysis of first data and second data and    │
│  conversion of the analyzed data into data related │ ～1030
│        to the rotations of main wheels      │
└─────────────────────────────────────┘
                │
                ▼
┌─────────────────────────────────────┐
│       Analysis of the user's workout, based on the      │
│   data related to the rotation of the first main wheel and  │ ～1040
│ the data related to the rotation of the second main wheel │
└─────────────────────────────────────┘
                │
                ▼
            ( End )
```

FIG. 10B

Pulling both sides
0 m / 30 m

3/8

0.0 00 : 02 0.1 >
km/h m:s kcal

( a )

Twist
0 m / 10 m

7/10

0.0 00 : 05 0.1 >
km/h m:s kcal

( a )

Pushing left side
0 m / 10 m

2/10

0.0 00 : 06 0.0 >
km/h m:s kcal

( a )

FIG. 11A

```
                    ( Start )
                        │
                        ▼
┌──────────────────────────────────────────┐
│  Collection of user's workout analysis data │ ～1110
│             in training mode                │
└──────────────────────────────────────────┘
                        │
                        ▼
┌──────────────────────────────────────────┐
│       Evaluation of user's workout ability,  │ ～1120
│    based on the user's workout analysis data │
└──────────────────────────────────────────┘
                        │
                        ▼
┌──────────────────────────────────────────┐
│ Production of customized training mode using AI model, │ ～1130
│       based on the user's workout ability data │
└──────────────────────────────────────────┘
                        │
                        ▼
┌──────────────────────────────────────────┐
│   Customized training mode execution and feedback │ ～1140
└──────────────────────────────────────────┘
                        │
                        ▼
                    ( End )
```

FIG. 11B

Free training

Accumulated
distance

## 10 m

Left and right balance

| 51% | | 49% |

| km/h | mm:ss | kcal |
| 5.6 | 00:30 | 0.9 |

>

( Finish )  ( Pause )

FIG. 11C

FIG. 12A

```
            ( Start )
               │
               ▼
┌───────────────────────────────────────────┐
│                                           │
│               Game mode                   │ ～1210
│                                           │
└───────────────────────────────────────────┘
               │
               ▼
┌───────────────────────────────────────────┐
│ Acquisition of first data and second data from a treadmill │ ～1220
│  for a wheelchair through a communication module           │
└───────────────────────────────────────────┘
               │
               ▼
┌───────────────────────────────────────────┐
│ Analysis of first data and second data and conversion │
│  of the analyzed data into data related to the        │ ～1230
│             rotations of main wheels                  │
└───────────────────────────────────────────┘
               │
               ▼
┌───────────────────────────────────────────┐
│ Matching the data related to the first main wheel │
│  and the data related to the second main wheel    │ ～1240
│              to an input command                  │
└───────────────────────────────────────────┘
               │
               ▼
┌───────────────────────────────────────────┐
│      Control of game application according      │ ～1250
│            to the input command                 │
└───────────────────────────────────────────┘
               │
               ▼
            ( End )
```

FIG. 12B

FIG. 12C

Europäisches
Patentamt

European
Patent Office

Office européen
des brevets

# EUROPEAN SEARCH REPORT

**Application Number**

EP 24 20 6457

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 7 004 885 B1 (WU HONG-SHENG [TW] ET AL) 28 February 2006 (2006-02-28) * column 3, lines 24-44; column 3, lines 53-64; figures 1-2 * | 1-10 | INV. G06F3/01 A63B21/015 A63B71/00 A63B22/02 |
| X | US 2007/173392 A1 (STANFORD CHRISTOPHER STEPHEN R [US]) 26 July 2007 (2007-07-26) * the whole document * | 1-10 | A63B24/00 A63B71/06 |
| X | KR 102 527 513 B1 (AIRPASS [KR]) 3 May 2023 (2023-05-03) * paragraphs [0023], [0026], [0030], [0033]; claim 1 * | 1-10 | ADD. A63B69/16 |

TECHNICAL FIELDS
SEARCHED       (IPC)

A63B

The present search report has been drawn up for all claims

| Place of search | Date of completion of the search | Examiner |
|---|---|---|
| Munich | 24 February 2025 | Vesin, Stéphane |

CATEGORY OF CITED DOCUMENTS

X : particularly relevant if taken alone
Y : particularly relevant if combined with another
　　document of the same category
A : technological background
O : non-written disclosure
P : intermediate document

T : theory or principle underlying the invention
E : earlier patent document, but published on, or
　　after the filing date
D : document cited in the application
L : document cited for other reasons

 

& : member of the same patent family, corresponding
　　document

EPO FORM 1503 03.82 (P04C01)

## ANNEX TO THE EUROPEAN SEARCH REPORT
## ON EUROPEAN PATENT APPLICATION NO.

EP 24 20 6457

This annex lists the patent family members relating to the patent documents cited in the above-mentioned European search report.
The members are as contained in the European Patent Office EDP file on
The European Patent Office is in no way liable for these particulars which are merely given for the purpose of information.

24-02-2025

| Patent document cited in search report | | Publication date | Patent family member(s) | | Publication date |
|---|---|---|---|---|---|
| US 7004885 | B1 | 28-02-2006 | NONE | | |
| US 2007173392 | A1 | 26-07-2007 | US 2007173392 A1 | | 26-07-2007 |
| | | | WO 2007087514 A1 | | 02-08-2007 |
| KR 102527513 | B1 | 03-05-2023 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230162389

- KR 1020240077789